(19) **Europäisches Patentamt / European Patent Office / Office européen des brevets**

(11) **EP 1 869 150 B2**

(12) # NEW EUROPEAN PATENT SPECIFICATION
After opposition procedure

(45) Date of publication and mention
of the opposition decision:
**31.08.2016 Bulletin 2016/35**

(45) Mention of the grant of the patent:
**30.05.2012 Bulletin 2012/22**

(21) Application number: **06726764.1**

(22) Date of filing: **13.04.2006**

(51) Int Cl.:
***C11B 9/00*** *(2006.01)*

(86) International application number:
**PCT/GB2006/001365**

(87) International publication number:
**WO 2006/109078 (19.10.2006 Gazette 2006/42)**

(54) **PERFUME COMPOSITIONS**

*PARFÜM-ZUSAMMENSETZUNGEN*

COMPOSITIONS DE PARFUMS

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.04.2005 GB 0507520**
**14.04.2005 GB 0507521**

(43) Date of publication of application:
**26.12.2007 Bulletin 2007/52**

(73) Proprietor: **Givaudan S.A.**
**1214 Vernier (CH)**

(72) Inventors:
• **BRADSHAW, David Jonathan**
**Ashford Kent, TN24 9RP (GB)**
• **PROVAN, Alan, Forbes**
**Kingsnorth, Ashford Kent, TN23 3GR (GB)**
• **IOANNOU, Christopher, James**
**Folkestone Kent, CT20 2LD (GB)**
• **BEHAN, John Martin**
**Ashford Kent TN25 4JB (GB)**
• **PERRING, Keith, Douglas**
**Ashford Kent TN24 8HS (GB)**

(74) Representative: **Givaudan Patents**
**Givaudan Schweiz AG**
**Global Patents**
**Ueberlandstrasse 138**
**8600 Dübendorf (CH)**

(56) References cited:
EP-A- 1 238 650   WO-A-00/57699
WO-A-2004/073670   US-B1- 6 585 961

• **MORRIS J A ET AL: "ANTIMICROBIAL ACTIVITY OF AROMA CHEMICALS AND ESSENTIAL OILS" JOURNAL OF THE AMERICAN OIL CHEMISTS' SOCIETY, AOCS PRESS, CHAMPAIGN, IL, US, 1 May 1979 (1979-05-01), pages 595-603, XP000645444 ISSN: 0003-021X cited in the application**
• **O. SECONDINI, DR.: 'Handbook of Perfumes and Flavors', 1990, CHEMICAL PUBLISHING CO., INC., NEW YORK pages 209-213 - 225,239,259,402**
• **S. ARCTANDER: 'Perfume and Flavor Chemicals (Aroma Chemicals) II', 1994, ALLURED PUBLISHING CORPORATION, USA pages 2661,2662 - 2605-2697**
• **S. ARCTANDER: 'Perfume and Flavor Chemicals (Aroma Chemicals) II', 1994, ALLURE PUBLISHING CORPORATION, USA pages 2661,2662 - 2605-2697**
• **W. A. POUCHER: 'Perfumes, Cosmetics and Soaps', vol. 1, 1959, CHAPMAN AND HALL LTD, LONDON pages 172,173 - 224-226**

**Description**

Field of the Invention

This invention relates to perfume compositions including flavour compositions. For the purposes of this invention a perfume composition is defined as a mixture of perfume ingredients, including if desired a suitable perfume solvent or solvents and optionally mixed with a solid substrate. Perfume ingredients are well known to those skilled in the art, and include those mentioned, for example, in S. Arctander, Perfume and Flavor Chemicals (Montclair, NJ. , 1969), in S. Arctander, Perfume and Flavor Materials of Natural Origin (Elizabeth, N.J., 1960) and in "Flavor and Fragrance Materials -1991 ", Allured Publishing Co. Wheaton, III. USA. Perfume ingredients may include natural products such as extracts, essential oils, absolutes, resinoids, resins, concretes etc., and also synthetic basic substances such as hydrocarbons, alcohols, aldehydes, ketones, ethers, acids, esters, acetals, ketals, nitrites, etc., including saturated and unsaturated compounds, aliphatic, arbocyclic and heterocyclic compounds. The invention is particularly concerned with perfume compositions that have the ability to detach or remove biofilms from surfaces.

Background to the Invention

Biofilms are collections of microorganisms (bacteria, fungi etc.) that are attached, directly or indirectly, to a solid phase abiotic or biotic surface. Biofilms grow in situ, and are usually embedded in extracellular polymeric substance (EPS) matrix of microbial or other origin. Biofilms are generally difficult to remove from a surface. The majority of bacteria in nature exist in biofilms, and biofilms often represent the "preferred" mode of growth for bacteria (Costerton JW, Lewandowski Z, Caldwell DE, Korber DR, Lappin- Scott HM (1995). Microbial biofilms. Annual Reviews of Microbiology Volume 49, pp. 711-745). Biofilms may be composed of only one type of microorganism, but frequently contain several types of microorganism living in a complex community. Biofilm communities can include bacteria, fungi, yeasts, protozoa, and other microorganisms. In fact, almost every surface exposed to liquids and nutrients can be colonised by microorganisms. Biofilms include those associated with animals (e.g. dental plaque in the mouth, bacteria on mucosal surfaces in the gut, or on the skin); pathogens that infect burns, wounds or implanted devices. Biofilms are also significant in a number of industrial settings, such as water and food processing systems. Biofilms are also a part of domestic life: in the home, biofilms are found in bathrooms, toilets, showers and kitchens. Biofilms are also of interest because they display a range of unusual properties - notably resistance to disinfection and removal. A great number of medical, industrial and domestic products aim to control biofilms, and many of these are fragranced.
Initially, the formation of a biofilm typically involves the adsorption of an organic monolayer ("conditioning film") to a surface that can change the chemical and physical properties of the surface. These adsorbed materials condition the surface and allow free- floating, pioneer microorganisms that encounter the conditioned surface to form a reversible, sometimes transient attachment. This attachment is influenced by electrical charges carried on the microorganism and by Van der Waals forces and by electrostatic attraction. Further chemical and physical structures can transform the reversible attachment to a permanent and essentially "irreversible" attachment, via adhesins on the surface of the microorganism and receptors on the surface. Following this irreversible attachment, the .production of extracellular polymeric substance can allow entrapment of other materials (such as organic materials, dead- cells and precipitated minerals etc.). These can add to the bulk and diversity of the biofilm. Pioneer attached bacteria may grow. Secondary colonizing bacteria may attach to the pioneer bacteria, and a mature biofilm community may develop.
Biofilms may be found on the surfaces of water tanks, pipes, surgical apparatus, food- processing vessels, etc., where the bacteria adhere tenaciously, resisting removal by washing and also gaining protection from antimicrobial compounds such as common disinfectants which cannot easily penetrate the polysaccharide matrix. Biofilms can act as a source of infections. Examples include seeding water systems with Legionella bacteria, leading to Legionnaire's disease; in a medical setting, biofilms may be responsible for a range of life-threatening infections associated with burns or wounds, or with prosthetic devices such as heart valves, catheters and replacement joints; hospital equipment (e.g. artificial respirators and ventilators) can become colonized with bacteria, leading to infection of vulnerable patients.
A further example of a biofilm is the dental plaque found on teeth, which is the causative agent of dental caries when bacteria such as *Streptococcus mutans* degrade sugars to organic acids, or on oral mucosal surfaces where the dental plaque biofilm is the aetiological agent which can lead to the development periodontal (gum) diseases.
Biofilms can colonize many household surfaces, including toilets, sinks, countertops, and cutting boards in the kitchen and almost all of the various surfaces hi bathrooms. These biofilms can act as a source of malodours, cause staining and may pose an infection hazard.
In industry, biofilms are responsible for enormous economic costs in lost industrial productivity and both product and capital equipment damage each year. For example, biofilms are notorious for causing pipe plugging, inefficiency of heat exchange systems, corrosion of metal surfaces and water contamination.
Biofilm contamination and fouling occurs in nearly every industrial water-based process, including food and drink production, water treatment and distribution, pulp and paper manufacturing, and the operation of cooling towers.

The most notable property of biofilms is their unusual resistance to a wide range of antimicrobial agents, which renders them difficult to disinfect. This inherent high resistance of biofilms is derived from a number of factors. The extracellular polymeric substance around biofilms may act as a barrier to diffusion of antimicrobials, as a food reserve and can prevent desiccation. Certain bacteria can secrete enzymes that inactivate antimicrobials; other bacteria, growing in the close proximity that a biofilm allows, which otherwise would be sensitive to the antimicrobial, can themselves be rendered "resistant" when in a biofilm. Furthermore, evidence is growing that microorganisms in biofilms can display resistant phenotypes, resulting from altered patterns of gene expression and inter- bacterial communication. These potential mechanisms of biofilm resistance have been described in several recent reviews (for example see Gilbert P, Maira-Litran T, McBain AJ, Rickard AH, Whyte FW. (2002). The physiology and collective recalcitrance of microbial biofilm communities. Advances in Microbial Physiology. Volume 46, pp. 202- 256).

Many products are designed, either directly or indirectly, to combat biofilms or biofilm- related problems. Such products generally involve some form of physical activity, optionally aided by cleaning materials such as surfactants, to detach the biofilm. Sometimes, these products also contain strong disinfectants, such as bleach or other antimicrobial actives, to kill or neutralize the microorganisms within the biofilm.

US Patent 6 585 961 B1 describes biofilm removal compositions containing antimicrobial essential oils.

Summary of the Invention

The present invention is based on the surprising discovery that certain perfume ingredients, individually or in combination, possess hitherto unknown biofilm-detachment properties. Such ingredients may be used to supplement the biofilm-detachment properties of a variety of products, for example general purpose cleaners.

We have carried out extensive testing of a large number of individual perfume ingredients and mixtures thereof to determine their effectiveness in detaching biofilms. -This testing has enabled us to classify perfume ingredients into different categories, namely those that assist biofilm detachment (referred to herein as Group A materials), those that are non- active to biofilm detachment (referred to herein as Group B materials), and those that are detrimental or antagonistic to biofilm detachment (referred to herein as Group C materials). Complete detachment of biofilms is not essential, with partial removal of biofilms being sufficient. Based on this classification of perfume ingredients, the invention enables the formulation of perfume compositions that are effective in assisting biofilm detachment while allowing a degree of freedom in formulation that permits consideration of the hedonic properties of the composition. The invention can thus enable formulation of perfume compositions that are effective in detaching biofilms and that also have good hedonic properties which can be tailored to suit a range of different product types.

In particular, a standardised biofilm detachment assay was used to quantify perfume ingredients in biofilm removal in terms of percent detachment values (PDVs). The standardised biofilm detachment assay is based on testing perfume compositions of 4 perfume ingredients present in equal amounts by weight, comprising 3 ingredients that fall in Group B and are non-active with regard to biofilm detachment (3,7-dimethyloct-6-en-1-ol; I-ethenyl-l,5-dimethylhex-4-enyl acetate; and phenylmethyl-2-hydroxybenzoate) in admixture of a perfume ingredient under test. Each 4 component perfume mixture was used at a concentration of 0.25% w/w in a quarter strength general purpose cleaner (GPC) base test mixture having the composition of Formula A as set out in Example 1(a) below. The test mixtures were tested by the procedure set out in Example I(a) below to determine a PDV for the perfume composition under test, and hence a PDV of the particular 4th perfume ingredient under test in the mixture indicative of the biofilm removal efficacy of that ingredient. PDVs for the perfume ingredients obtained in this way were rounded to the nearest integer. References in this specification to percent detachment value or PDV of a material means a value obtained using this standardised biofilm detachment assay, as defined above.

Group A materials that assist biofilm detachment are defined as those having a PDV (as defined above) of at least 9. Group B materials that are non-active to biofilm detachment are defined as those having a PDV (as defined above) of less than 9 and greater than 0. Group C materials that are antagonistic to biofilm detachment are defined as those having a PDV (as defined above) of less than or equal to 0.

There is provided a perfume composition comprising at least 15% by weight of at least three Group A perfume ingredients and at least one Group B ingredient, and wherein Group A and Group B ingredients together constitute at least 80% by weight of the perfume composition, wherein the Group A ingredients are selected from the group consisting of

(2E)-tridec-2-enenitrile
(2Z)-2-ethyl-4-(2,2,3-trim ethylcyclopent-3-en-1-yl)but-2-en-1-ol
(3E)-3-methyl-4-(2,6,6-trimethylcyclohex-2-en-1-yl)but-3-en-2-one
1-(2,3,8,8-tetramethyl-1,2,3,5,6,7,8,8a-octahydronaphthalen-2-yl)ethanone
1-(2,6,6,8-tetramethyl-tricyclo [5.3.1.0^ {1,5}]undec-8~en-9-yl)ethanone
1-(methyloxy)-4-[(1E)-prop-1-enyl]benzene

1,4-dioxacycloheptadecane-5,17-dione

1-[1,1,2,6-tetramethyl-3-(1-methylethyl)-2,3-dihydro-1H-inden-5-yl]ethanone

1-methyl-4-(I-methylethyl)-2-[(1E)-prop-1-enyl]benzene

1-methylethyl tetradecanoate

2-(2-methylpropyl)-4-hydroxy-4-methyl-tetrahydropyran

2,6,10-trimethylundec-9-enal

2- [2-(4-methylcyclohex-3-en-1-yl)propyl] cyclopentanone

2-methylundecanal

5-methyl-2-(1-methylethyl)phenol

cinnamic alcohol

clove bud rectified extra DQ P353

cyclohexadecanolide

cyclopentadecanone

decanol DQ

dodecanenitrile

eucalyptol

eucalyptus globulus

eugenol rectified

ginger oils

isoamyl acetate

nonanol

patchouli oil

phenylmethanol

propane-I,2-diol

tea tree oil DQ

tributyl-2-(acetyloxy)propane-1,2,3-tricarboxylate

1H-indole

(2E)-2-pentyl-3-phenylprop-2-enal

(4E)-4-tricyclo[5.2.1.0^{2,6}]dec-8-ylidenebutanal

2,6,6,8-tetramethyltricyclo[5.3.1.0^{I,5}]undec-8-yl acetate

2-[(2-{[2-(methyloxy)propyl] oxy }propyl)oxy]propan-1-ol

2~methyldecanenitrile

1-(1,1,2,3,3,6-hexamethyl-2,3-dihydro- 1H-inden-5-yl)ethanone

1-(3,5,5,6,8,8-hexamethyl-5,6,7,8-tetrahydronaphthalen-2-yl)ethanone

4-(1,7,7-trimethylbicyclo[2.2.1]hept-2-yl)cyclohexanol

Silvanone (Silvanone is a Trade Mark)

2-heptylcyclopentanone

3 -methyldodecanenitrile

(6E)-3,7,11 -trimethyldodeca-1 ,6,10-trien-3-ol

methyl (2E)-non-2-enoate

(2E)-2-hexyl-3-phenylprop-2-enal

ethyl 3-(1-methylethyl)bicyclo[2.2.1]hept-5-ene-2-carboxylate

3-methylcyclopentadecanone

ylang ylang oil

2-(1,1-dimethylethyl)cyclohexyl acetate.

2,4-dimethyl-4,4a,5,9b-tetrahydroindeno[ 1 ,2- {d}] [1,3]dioxine

4,6,6,7,8,8-hexamethyl-I,3,4,6,7,8-hexahydroindeno[5,6-{c}]pyran (e.g. in the form of Galaxolide - Galaxolide is .a Trade Mark)

[4-(1-methylethyl)cyclohexyl]methanol

capsicum oleoresin DQ

lime terpeneless DQ

citronella Ceylon DQ

rose flavour base ABF0339A

marjoram French DQ

jasmin absolute DQ

coriander DQ

lavender oil DQ

iso propyl alcohol DQ

amyl cinnamic aldehyde DQ
amyl caproate DQ

ketones of general formula RCOR' having an octanol-water partition coefficient of at least 4 (expressed as. a logarithm to base 10), where R and R' are independently hydrocarbyl residues that may be aliphatic or aromatic, saturated or unsaturated, and combinations thereof, but may not contain, other functional groups, including

1-(2,3,8,8-tetramethyl-1,2,3,4,5,6,7,8-octahydronaphthalen-2-yl)ethanone (log P = 5.28)
1-(2,6,6,8-tetramethyltricyclo[5.3.1.0^{1,5}undec-8-en-9-yl)ethanone (log P = 5.17)
1-(1,1,2,3,3,6-hexamethyl-2,3-dihydro-1H-inden-5-yl)ethanone (log P = 5.80)
1-(3,5,5,6,8,8-hexamethyl-5,6,7,8-tetrahydronaphthalen-2-yl)ethanone (log P = 6.37)
3-methylcyclopentadecanone (log P = 6.33)
1-[1,1,2,6-tetramethyl-3-(1-methylethyl)-2,3-dihydro-1H-inden-5-yl]ethanone (log P = 6.14)

and Group B ingredients are selected from the group consisting of

(3Z)-hex-3-en-1-ol
1,7,7-trimethylbicyclo[2.2.1]hept-2-yl acetate
1-methyl-4-(1-methylethylidene)cyclohex-1-ene
2-(phenyloxy)ethanol
2,6,10-trimethyl-1-acetyl-cyclododeca-2,5,9-triene
2-{[2-(ethyloxy)ethyl]oxy}ethanol
3,7-dimethyloct-6-en-1-ol
diethyl benzene-1,2-dicarboxylate
(4E)-dec-4-enal
ethyl-2-methyl-1,3-dioxolan-2-yl acetate
5-hepxyldihydrofuran-2(3H)-one
origanum
1 -(methyloxy)propan-2-ol
2,2-dimethyl-3-(3-methylphenyl)propan-1-ol
tricyclo[5.2.1.0^{2,6}]dec-4-en-8-yl propanoate
3a,6,6,9a-tetramethyldodecahydronaphthol [2,1 -{b}]furan
hexyl 2-hydroxybenzoate
phenylmethyl-2-hydroxybenzoate
cyclohexadec-5-en-1-one
Rose fragrance 0409(TM) (available from Quest International)
1-ethenyl-1,5-dimethylhex-4-enyl acetate
(4Z)-dec-4-enal
1-(5,5-dimethylcyclohex-1-en-1-yl)pent-4-en-1-one
3-methyl-1-(2-methylpropyl)butyl acetate
2-(methyloxy)-4-propylphenol
2-(acetyloxy)-1-[(acetyloxy)methyl] ethyl acetate
Thyme Red
4-(1,1 -dimethylethyl)cyclohexyl acetate
5-methyl-2-(1-methylethyl)cyclohexanol
Aldehyde C10 DQ
Lime Terpenes Washed DQ
Blackcurrant Base ABF0972
Grapefruit DQ
Parsley Herb
Orange Terpenes Ex Concentrate DQ
Iso Amyl Butyrate DQ
Cinnamon Supra ABF1092
Cardamom English Distilled DQ
Apple Base ABF1016
Orange Terpeneless DQ

DQ = Dental Quality

Q = Quest International

The octanol-water partition coefficient (P) of a material i.e. the ratio of a material's equilibrium concentration in octanol and water, is well known in the literature as a measure of hydrophobicity and water solubility (see Hansen and Leo, Chemical Reviews, 526 to 616, (1971), 71; Hansch, Quinlan and Lawrence, J. Organic Chemistry, 347 to 350 (1968), 33). High partition coefficient values are more conveniently given in the form of their logarithm to the base 10, log P. While log P values can be measured experimentally i.e. directly, and measured log P data is available for many perfumes, log P values are most conveniently calculated or approximately estimated using mathematical algorithms. There are several recognised calculation or estimation methods available commercially and/or described in the literature (see for example A Leo, Chem. Rev 93(4), 1281-1306, (1993), " Calculating log P oct from structures"). Generally these models correlate highly but may for specific materials produce log P values which differ in absolute terms (by up to 0.5 log units or even more). However, no one model is universally accepted as the most accurate across all compounds.

This is particularly true for estimates on materials of high log P (say 4 or greater). In the present specification, log P values are obtained using the estimation software commercially available as 'Log P' from Toronto-based Advanced Chemistry Development Inc (ACD) which is well-known to the scientific community, and accepted as providing high-quality predictions of log P values. References to log P values thus mean values obtained using the ACD software.

In the lists of Group A and Group B materials, those marked with the symbol are known to have some antimicrobial (bacteriostatic or bactericidal) properties. See, for example, the paper by Morris J A, Khettry A, Seitz E W (1979) Antimicrobial activity of aroma chemicals and essential oils. Journal of the American Oil Chemistry Society Volume 56, pp. 595-603; and WO 01/24769 of Firmenich & Cie.

The perfume composition may comprise at least 5 or at least 6 Group A ingredients.

The use of a mixture of several Group A ingredients assists formulation of compositions having desired hedonic properties as well as biofilm detaching properties. The perfume composition may comprise at least 25% by weight, at least 30% by weight, at least 40% by weight or possibly at least 60% by weight of Group A perfume ingredients.

The perfume composition desirably includes at least one, preferably at least two or at least three or more Group B perfume ingredients, as defined above, i.e. having a PDV between 0 and 9 determined by the standardised biofilm detachment assay defined herein. These materials are not detrimental to the biofilm detaching properties of the composition and so enhance the scope for formulation of compositions having desired hedonic properties without having an adverse effect on biofilm detachment.

A preferred perfume composition comprises at least 3 Group A perfume ingredients and at least 3 Group B perfume ingredients.

The perfume composition should preferably not include any Group C perfume ingredients or should keep these to a minimum, as these will have an adverse effect on biofilm detachment properties of the composition. Group C ingredients are as defined above, i.e. having a PDV less than or equal to 0 determined by the standardised biofilm detachment assay defined herein. Perfume compositions in accordance with the invention comprise at most 20 wt % Group C ingredients. Preferably they comprise at most 15 wt %, at most 10 wt % or at most 5 wt % or even at most 3 wt % Group C ingredients.

Particular examples of Group C materials are

2-methyl-3-[4-(methyoxy) phenyl] propanal
(2E)-3-phenylprop-2-enal
lemongrass oil
1,3-dimethylbut-3-enyl 2-methylpropanoate
Muguet base AB7001 (TM) (Q)
Moss base AB7004(TM) (Q)
Sandalone AC802(TM) (Q)
Jasmin AB7002(TM) (Q)
prop-2-enyl [(2-methylbutyl)oxy] acetate
2,6-dimethylhept-5-en-2-ol
phenylmethyl acetate
Carvone
3-pentyltetrahydro-2H-pyran-4-yl acetate
3 ,7-dimethylocta-1,6-dien-3-ol
4-(4-hydroxy-4-methylpentyl)cyclohex-3-ene-1-carbaldehyde
methyl-(3-oxo-2-pentylcyclopentyl)acetate
3-methyl-5-phenylpentan-1-ol
(1 E)-1-(2,6,6-trimethylcyclohex-2-en-1-yl)pent-1-en-3-one
Orange oil

2-phenylethanol
2-(4-methylcyclohex-3-en-1-yl)propan-2-ol
1-[(2-hydroxypropyl)oxy]propan-2-ol
(6Z)-3,7-dimethylnona-1,6-dien-3-ol

Q = Materials available from Quest International

Each of the Group A ingredients, and the Group B ingredients if present, is preferably present in an amount not exceeding 20% by weight, more preferably not exceeding 15% by weight and most preferably not exceeding 10% by weight.

Preferred ingredients of Groups A and B are those that deliver effective odour intensities in use, and are characterised by exhibiting odour threshold levels in air of lower than 2000 ppb (parts per billion) by weight, or even more preferably lower than 1000 ppb, and yet more preferably lower than 500 ppb. Odour thresholds are discussed and examples provided hi many literature sources, among which may be mentioned in particular: "Standardized Human Olfactory Thresholds" , M Devos et al, ERL Press at Oxford University Press (1990), and "Compilation of Odor and Taste Threshold Values Data" , F A Fazzalari (editor), ASTM Data Series DS 48A (1978).

The perfume composition desirably includes one or more perfume ingredients with antimicrobial properties if such properties are desired, such as those materials identified by the symbol ^ in the lists above. This invention concerns perfume compositions that have the ability to detach (i.e. interrupt and end the attachment of) biofilms that comprise, for example, mould, fungi, algae and/or bacteria, from surfaces and a process whereby an effective amount of the composition is applied to the surface and/or the biofilm(s) attached to the surface.

The biofilm-detaching perfume compositions of this invention desirably have the following characteristics:

(1) do not leave a film under which microorganisms may remain viable.
(2) are effective against a wide spectrum of microorganisms.
(3) negate and retard the overgrowth of microorganisms.
(4) prevent or retard biofilm formation.
(5) may potentiate the biofilm detachment properties of bases and do not retard base activity.

The mode of action of this invention is not systemic. The mode of action is universal in nature, so that the overgrowth of any microbial species may be prevented.

The compositions of the invention are effective against biofilms on all types of solid or semi-solid surfaces, as can be found e.g. in a household such as, glass, stone, wood, metal, plastic, fabrics, gauze, ceramic, porcelain and animal or human tissue (e.g. a skin surface or an oral surface), vegetable matter and the like.

The compositions of this invention are effective against microorganisms, such as, mould, fungi, algae, yeast, bacteria (Gram-negative and Gram-positive) and viruses.

The modes of action of the biofilm-detaching compositions of this invention are not necessarily bacteriostatic or bactericidal or antibacterial or the like, in the normal sense of such terms. Instead, the compositions detach microorganisms and bring about a reduction in the populations of microorganisms on the surface. However, the compositions of the invention can be so designed, if required, to deliver explicit antimicrobial effects (e.g. bacteriostatic, bactericidal etc), in addition to the biofilm removing effect. Alternatively, compositions with low or no inherent antimicrobial activity may also be produced.

The perfume compositions of the present invention may be incorporated into a wide variety of consumer products, particularly personal, domestic and industrial products, to provide not only desirable odour properties but also to deliver or enhance the biofilm- detachment properties of such products. The consumer products may be in a range of the different physical forms, including slurries, suspensions, solutions, pastes, emulsions, etc.

In particular, the compositions of the invention may find application in all types of cleaning products (household, medical, industrial), particularly hard surface cleaning products. For example, they could be used in general-purpose-cleaners or all purpose cleaners, dishwashing products (either hand- or machine-dishwashing), or cleaners for kitchen surfaces such as sinks, worktops, ovens, utensils; cleaners for all types of domestic floors such as tiles, wood, carpets, cement and linoleum; they could be useful in cleaners for domestic appliances such as washing machines, dishwashers, food processors, vacuum cleaners and the like; in the bathroom they could find application in cleaners for showers, baths, taps, plugs, drains, toilets, bidets, washbasins, surrounding tiles, shower curtains and doors; for toilet products they could also find application in under-the-rim products (rim liquids, rim blocks), in-tank products (blocks and liquids), on-tank products; in the wider domestic environment they may find application in car, shoe/boot, window, wall, ceiling and other miscellaneous cleaning products; they may be applied in a range of household polishing products; outside the home they may find application in cleaners or treatments for concrete, tarmac, pipelines, wooden surfaces, brickwork, garden furniture, domestic ponds, alloy wheels and metals, glass, fountains and the like. In medical and industrial settings they may find application in a wide variety of cleaning products with possibly different product delivery mechanisms in the form of fragrance cartridges, multi- nozzle dispensing containers etc.

Additionally they may find application in a range of personal care, hair care, skin care and cosmetic products. For example, they may be used in face creams, lipsticks, makeup/nail removers, sun creams, hand and body washing products. The composition of this invention can be used as a deodorant. It can be directly applied as a liquid, or can be placed on an absorbent pad or the like and applied in liquid form by putting on the affected surface. Adhesion of microbes, that directly or indirectly cause body odours, is prevented or retarded, and such microbes are detached. The compositions and methods of this invention may be useful also in the treatment of dermatitis and itching caused by microbes, to coat first-aid products (e.g., gauze pads, band-aids, etc.), to treat athlete's feet, in cosmetic preparation (e.g., base lotion, eye mascara, etc.), for washing food and meat cases, food counters and the like, to treat cuts, scratches and the like, as an eye wash, to treat dandruff, treatment of acne, to clean contact lenses, shaving creams, hand creams, and after shave lotions, ear wax removal, in aerosol sprays, in a liquid dentifrice, as a disinfectant and in shampoos.

Furthermore, the compositions of the invention can find application in dental care products such as dentifrices, denture care products, mouthwashes, breath freshening powders/tablets, breath films, teeth whitening products, chewing gums (where the term "chewing gum" is intended also to encompass bubble gum), dental floss, dissolvable mouth films, lozenges, gels, mousse, creams, aerosols (breath sprays) etc. to assist in the removal of dental plaque, and contribute in this way to dental hygiene. The compositions of the invention may also be used in oral care products. Also included are over the counter or prescription products for the prevention or treatment of gingivitis, plaque, tartar, caries and oral malodour.

The appropriate effective dosage of perfume composition in each product will depend upon the nature and purpose of the product. For example, in a window cleaner product the minimum useful dosage of a perfume composition of the invention is 0.05 % by weight, more preferably 0.1 % or 0.2% by weight, whereas in a rim-block toilet product the dosage would be much higher, around 5% or 10% by weight, or even higher. Suitable levels of perfume composition in different product types can be readily determined, and are known to those skilled in the art.

The invention also covers use of a perfume ingredient in Group A for the purpose of detaching a biofilm. Preferred features of this aspect are as discussed above in connection with the perfume composition of the invention.

The perfume composition is conveniently present in a consumer product, e.g. as discussed above. The composition or product can be readily applied to a surface in known manner, appropriate to the form and type of the composition or product. Appropriate dosages of composition or product and treatment times can be readily determined by one skilled in the art.

Also within the scope of the invention is a method of detaching a biofilm from a surface, comprising application to the surface of a perfume composition or perfumed product in accordance with the invention. The treatment can be repeated as required, for long term effectiveness.

The invention will be further described, by way of illustration, in the following Examples and with reference to the accompanying Figures, in which:

Figure 1 is a graph of percent detachment value (PDV) versus percent active (Group A)/antagonist (Group C) material in perfume compositions as tested in Example 5;

Figure 2 is a graph of percent detachment value (PDV) versus percent antagonist (Group C) material in perfume compositions as tested in Example 6; and Figure 3 is a bar chart of percent detachment value (PDV) for perfume compositions in water as tested in Example 7.

Examples

Example 1 Biofilm-Detachment and Kill Assays

Example 1(a): Biofilm Detachment

A bacterial culture of the micro-organism *Escherichia coli* ATCC 10536 (American Type Culture Collection (ATCC), P.O. Box 1549, Manassas, VA 20108, USA) was grown overnight at 37°C in Tryptone Soya Broth (TSB) (Oxoid, Basingstoke, UK). The E. coli suspension was diluted a thousand fold in TSB and 150 $\mu$l of this dilution was then added to each well in a 96 well microtitre plate (Sterilin, Staffs, UK). The microtitre plate lids were then tape-sealed and incubated at 370°C,static. After 18 hours incubation, the bacterial suspension was discarded from each well followed by the addition of 200 $\mu$l sterile de-ionised water. This step represented a wash cycle and a total of two of these wash cycles were conducted. In this way, test biofilms remaining attached were produced within the wells.

Perfume compositions comprising perfume ingredients to be tested were prepared and the compositions added to a high impact general purpose cleaner base (diluted to quarter strength) together with de-ionised water and the surfactant Synperonic 91/10 (Synperonic is a Trade Mark) to produce test mixtures with the composition as set out in Formula A below. (Synperonic 91/10 is a mixture of ethoxylated alcohols averaging C9-C11 (10 EOs)) that functions as a solubiliser for the perfume ingredients to aid fragrance solubility.

Formula A:

0.25 %w/w perfume composition 0.5 %w/w Synperonic 91/10 (85%wlw) (Uniqema, Wirral, UK)
2.25 % w/w de-ionised water
97%w/w High Impact GPC base diluted with water to quarter strength, the composition of which (after dilution to quarter strength) is as follows:

| | |
|---|---|
| 1% w/w | Texapon NSO/IS |
| 0.25% w/w | Ethanol (96%) |
| 0.05% w/w | Bronidox L |
| 95.7% w/w | Water (demineralised) |

Texapon NSO/IS comprises sodium lauryl ether sulphate (28 %) and is available from Henkel, Hertfordshire, UK (Texapon is a Trade Mark). Bronidox L comprises 5-bromo- 5-nitro-1.3 dioxane available from Henkel, Hertfordshire, UK (Bronidox is a Trade Mark).

200 $\mu$l of each test mixture was added to a total of eight wells, representing a column in the microtitre plate. Controls included a) formula A without perfume and with additional water in place of the perfume composition, and b) sodium lauryl sulphate (SLS) (2% w/w final concentration prepared in deionised water). Controls were added to separate microtitre plate columns. SLS is known to have biofilm detachment properties and is used as a positive control to provide an indication of the maximum level of detachment that could be obtained.

Water alone was added to a single column to act as a negative control. AU of the test mixtures and controls were left in the wells for 20 minutes, static, at 21 °C. The contents of each well were then discarded and two wash cycles conducted. Next, 200 $\mu$l of crystal violet stain solution (0.01 % w/w) was added and left static for 15 minutes at 21°C. Again the contents of each well were discarded and two wash cycles conducted. Finally, 5 the crystal violet was solubilised by adding 200 $\mu$l ethanol (70% w/w) to each well for 15 minutes at 21°C, static.

The crystal violet binds to any remaining biofilm, and is used to quantify the amount of biofilm remaining attached in the wells, and hence the amount of biofilm removal. The method used was based on the technique ) described in the paper by O'Toole G. A., Pratt L. A., Watnick P. L, Newman D. K., Weaver V. B., Kolter R. (1999) Genetic approaches to the study ofbiofilms. Methods in Enzymology, Vol. 310, pp. 91-109.

The crystal violet was quantified by measuring the optical density at 540 nm (OD540) of each well using a plate reader (model MRX, Dynatech laboratories, Chantilly, VA, USA). A percentage detachment value (PDV) for each test mixture was then calculated using the following formula:

$$PDV = 100\text{-}([OD_{540} \text{ for Test Mixture} / OD_{540} \text{ for Water Control}] \times 100).$$

Experiments showed that a test mixture comprising the ¼ strength base, Synperonic 91/10 and water but no perfume composition gave a maximum percentage detachment value of 20%. This was thus deducted from the PDV value determined as set at above to give Detachment Index (DI) for each perfume composition tested. The SLS positive control gave a PDV of about 80 %.

Using the procedure set out in Example 1(a), individual perfume ingredients and also perfume compositions containing mixtures of perfume ingredients prepared in quarter strength GPC base were investigated and the biofilm detaching properties of perfume ingredients and mixtures were determined. Using these results individual perfume ingredients falling into Groups A, B and C defined above were identified. Based on the results of these experiments, perfume compositions comprising mixtures of perfume ingredients with potentially good biofilm detaching properties were formulated and formulation ingredient studies were carried out. Test formulations were then further tested using the procedure of Example 1(b).

Example 1(b) Biofilm detachment - in-use simulation study

White ceramic tiles 38mm x 38mm were cleaned by submerging in ethanol (70% w/w) followed by a wash in de-ionised water and then towel dried. A culture of the microorganism Escherichia coli ATCC 10536 was grown overnight at 37°C in (TSB). The E. coli suspensi on was diluted a thousand fold in TSB and tiles were submerged in the bacterial suspension and incubated at 37°C, static.

After 18 hours incubation, the tiles were removed and placed in a series of glass Petri dishes with the test surface of each tile face up. A total of five tiles were added to each Petri dish. One Petri dish was prepared in this way per test ingredient or control. 100ml of test ingredients or compositions were added to each Petri dish and maintained at 24°C. at 60 revolutions per minute for 20 minutes in a shaker incubator (model 4628-IGMNPCE, Jencons, Bedfordshire, UK). In addition, controls included were sodium lauryl sulphate (0.28 % w/w), water, formula A without perfume and air only. After 20 minutes exposure, the test ingredient was discarded and the tiles washed in de-ionised water. Crystal violet (0.01 % w/w final concentration) was then added to the tiles for five minutes at 21 °C, static.

The tiles were then removed from the stain and washed in de-ionised water. The tiles were then allowed to dry and digital photographs were taken of the tiles. In addition, visual judgment using a score system of 0-5 was used to compare the degree of biofilm removal by the ingredients. A score of 0 represented a completely clean tile and 5 a tile covered with untreated biofilm as a control.

These investigations demonstrated that this invention can deliver measurable differences in activity on biofilm-soiled surfaces, and clearly show active ingredients that enhance the cleaning and/or disinfection of biofilms for hygiene, cleaning and oral care products.

Example 2

Biofilm-Detaching Composition A

A biofilm-detaching perfume composition in accordance with the present invention was prepared by mixing equal concentrations of six active (Group A) ingredients as listed below:

1-(methyloxy)-4-[(1E)-prop-1-enyl]benzene*
1-[1,1,2,6-tetramethyl-3-(1-methylethyl)-2,3-dihydro-1H-inden-5-yl]ethanone*
Dodecanenitrile*
(2E)-tridec-2-enenitrile*
2-(phenyloxy)ethanol
2,2-dimethyl-3-(3-methylphenyl)propan-1-0I)**

*Group A
**Group B

The perfume composition was used at a concentration of 0.25% w/w in the quarter strength GPC base composition of Formula A above, and was tested by the procedures of Examples 1(a) and I(b). This composition gave a mean DI value of 19% (n = 2) determined using the procedure set out in Example 1(a).

Example 3

The procedure described in example I(a) was repeated but wherein flavour compositions comprising flavour ingredients from the oral care palette to be tested were prepared. The compositions were added to a modified transparent silica toothpaste base (diluted to quarter strength) together with de-ionised water to produce test mixtures with the composition as set out in Formula B below.

Formula B:

0.25 %w/w     flavour composition
99.75 % w/w   Modified transparent silica toothpaste base diluted with water to quarter strength, the composition of which (after dilution to quarter strength) is as follows:

| | |
|---|---|
| 16.25% w/w | Sorbitol (70% w/w) Neosorb® |
| 0.062% w/w | sodium fluoride |
| 0.025% w/w | Tri sodium phosphate anhydrous |
| 1.0% w/w | PEG 1500 |

(continued)

| 0.2% w/w | Saccharin (25 % w/w solution prepared in de-ionised water) |
| to 100% w/w | Water (demineralised) |

Sorbitol is available from Roquette, France. Tri sodium phosphate anhydrous is available from ThermPhos, Oldbury, UE-. Polyethylene glycol (PEG) is available from Univar, Cheshire, UK. Saccharin is available from Ellis and .Everard, UK. Sodium fluoride is available from Sigma, Dorset, UK.

A percentage detachment value (PDV) for each test mixture was then calculated using the following formula:

$$PDV = 100\text{-}([OD_{540} \text{ for Test Mixture} / OD_{540} \text{ for Water Control}] \times 100).$$

Experiments showed that a test mixture comprising the ¼ strength modified transparent silica toothpaste base but no flavour composition gave a percentage detachment value of approximately -10% . The PDV scores were therefore adjusted to account for base influences on flavour behaviour and Detachment Index (DI) values quoted for each flavour composition. The SLS positive control gave a PDV of about 80 %.

Individual flavour ingredients and also flavour compositions containing mixtures of flavour ingredients prepared in quarter strength modified transparent silica toothpaste base were investigated and the biofilm detaching properties of flavour ingredients and mixtures were determined. Using these results individual flavour ingredients falling into Groups A, B and C defined above were identified. Based on the results of these experiments, flavour compositions comprising mixtures of flavour ingredients with potentially good biofilm detaching properties were formulated and formulation ingredient studies were carried out.

Biofilm detaching composition E

A biofilm-detaching flavour composition in accordance with the present invention was prepared by mixing equal concentrations of four active (Group A), one neutral (Group B) and one antagonist (Group C) ingredients as listed below:

Lavender oil*
Tea tree oil*
Peppermint arvensis co-distilled*
Coriander*
Origanum**
Lemongrass oil***

* Group A
**Group B
*** Group C

The flavour composition was used at a concentration of 0.25 % w/w in the quarter strength modified transparent silica toothpaste of Formula B above, and was tested by the procedure of Example 1(a). This composition gave a mean DI value of 31 % (for tests of 4 samples, i.e. n=4) determined using the procedure set out in Example l(a).

Biofilm detaching composition G

A biofilm-detaching flavour composition in accordance with the present invention was prepared by mixing equal concentrations of five active (Group A) ingredients and one non- active (Group B) ingredients as listed below:

Capsicum oleoresion*
Lime terpeneless*
Lavender oil*
Tea tree oil*
Anethole synthetic*
Blackcurrent base ABF 0972**

11

\*Group A
\*\* Group B

The flavour composition was used at a concentration of 0.25% w/w in the quarter strength modified transparent silica toothpaste of Formula B above, and was tested by the procedure of Example 1(a). This composition gave a mean DI value of 43 % (for tests of 4 samples, i.e. n=4) determined using the procedure set out in Example I(a).

Example 4

A biofilm-detaching composition in accordance with the present invention having both biofilm detachment and deodorant odour-reducing properties was prepared, having the following composition:

| Group | Ingredient | % by weight (total ingredients 100% w/w) |
|---|---|---|
| B | phenylmethyl 2-hydroxybenzoate | 25 |
| A | (3Z)-hex-3-enyl 2-hydroxybenzoate | 6 |
| B | 2-(methyloxy)-4 propylphenol | 4 |
| A | 1,4-dioxacycloheptadecane-5,17-dione | 6 |
| A | 1,4-dioxacycloheptadecane-5,17-dione | 6 |
| A | 4-methyl-2-(2-methylpropyl)tetrahydro-2H-pyran-4-ol | 22 |
| D | Geranium African Blk 0901 | 2 |
| D | 2-hexylcyclopent-2-en-1--one | 2 |
| A | 1-methylethyl tetradecanoate | 25.6 |
| B | Raspberry ketone 10 % CARB AA 2422 | 4 |
| D | Sage clary | 2.4 |
| A | 1-(2,6,6,8-tetramethyltricyclo[5.3.1.0^{1,5}]undec-8-en-9-yl)ethanone | 1 |
| | **Percent detachment value (PDV)** | **18** |
| | **5 hour % odour reduction score** | **22** |

D indicates that the material is not yet identified, but is tentatively allocated to group A or B.
The perfume composition was used at a final concentration of 1 % w/w in the quarter strength GPC base composition of Formula A, and was tested by procedure Example I(a) for biofilm detachment score. The result was adjusted to give a PDV value of 0 for the unperfumed composition.
The perfume composition embodying this invention was made and tested for deodorant action in an underarm product, using the deodorant value test generally as described in US-4-289641 using the formulation described in Formulation 1. The deo composition and the method for the deodorant value test are set out below.

Formulation 1 :

| Ingredient | Content (% by weight) |
|---|---|
| Quest fragrance | 1.0 |
| Isopropyl myristate | 1.0 |
| Propellant* 40 psig | 60.0 |
| Ethanol B | to 100.00 |
| *Hydrocarbon propellant. This can be any deodorised blend of n-propane, n-butane or Isobutane having a pressure of 40 pounds per square inch gauge or 2.812kg/cm$^2$ gauge (337 kPa). | |

The deodorant value test was carried out using a panel of Caucasian male subjects. A standard quantity (2 second spray) of an aerosol product containing the perfume composition or a soap control was applied to the axillae of the panel members in accordance with a statistical design.

Average scores for each test product and the control product were then determined and the score for each test product was subtracted from the score for the control product. A percent odour reduction score was determined for the test deo perfume.

Example 5

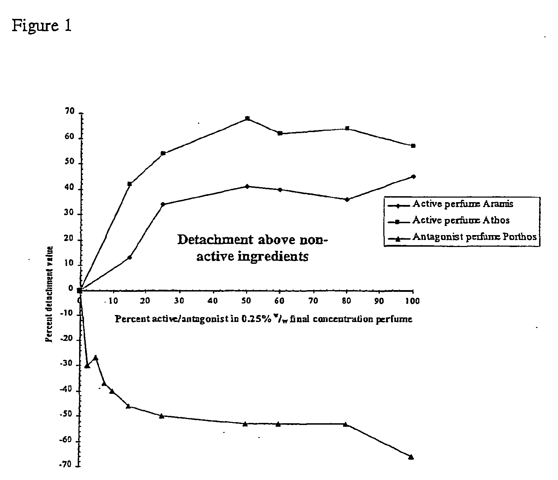

Perfume compositions containing a total of up to 15 ingredients at differing concentrations were selected from the active (Group A), non-active (Group B) and antagonist (Group C) ingredient list and formulated in the quarter strength GPC base composition of Formula A above. A non-active perfume composition contained 11 Group B ingredients. Two active perfume compositions called Aramis (Table 1) and Athos (Table 2) each additionally contained 4 Group A ingredients. An antagonist perfume composition called Porthos (Table 3) additionally contained 4 Group C ingredients. The final concentration of the perfume compositions as tested was 0.25 %w/w in GPC (Formula A), consistent with previous investigations. The percent active or antagonist ingredient in each perfume composition ranged from 0 to 100 percent with the remainder of the formulation containing non-active (Group B) perfume ingredients. The perfume compositions were tested using the microtitre plate technique as set out in Example 1(a) and a percent detachment value (PDV) calculated for each perfume composition. The results were adjusted to give a PDV value of 0 for the non-active perfume composition, containing Group B ingredients only. These results are presented in Tables 1 to 3 and in Figure 1 for the perfume formulations as listed below. In addition, a typical household cleaning product perfume (the composition of which is set out in Table 4) was also tested in the same way for biofilm detachment properties at a final concentration of 0.25 %w/w in the quarter strength GPC base of formula A.

**Table 1 Active perfume composition Aramis**

| Group | Ingredient | % by weight (total Ingredients 100% $^w/_w$) | | | | | | |
|---|---|---|---|---|---|---|---|---|
| | | 0 | 15 | 25 | 50 | 60 | 80 | 100 |
| B | Rose fragrance 0409™ (available from Quest International) | 11.1 | 9.4 | 8.3 | 5.6 | 4.4 | 2.2 | 0 |
| B | Ethyl-2-methyl-1,3-dioxolan-2-yl acetate | 5.6 | 4.7 | 4.2 | 2.8 | 2.2 | 1.1 | 0 |
| B | 5-heptyidihydrofuran-2(3H)-one | 2.8 | 2.4 | 2.1 | 1.4 | 1.1 | 0.6 | 0 |
| B | Origanum | 13.9 | 11.8 | 10.4 | 6.9 | 5.6 | 2.8 | 0 |
| B | 1-(methyloxy)propan-2-ol | 13.9 | 11.8 | 10.4 | 6.9 | 5.6 | 2.8 | 0 |
| B | 2,2-cimethyl-3-(3-methylphenyl)propan-1-ol | 8.3 | 7.1 | 6.3 | 4.2 | 3.3 | 1.7 | 0 |
| B | tricyclo[5.2.1.0^{2,6}]dec-4-en-8-yl propanoate | 13.9 | 11.8 | 10.4 | 6.9 | 5.6 | 2.8 | 0 |
| B | 1,7,7-Trimethylbicyclo[2.2.1]hept-2-yl acetate | 5.6 | 4.7 | 4.2 | 2.8 | 2.2 | 1.1 | 0 |
| B | 2-{[2-(Ethyloxy)ethyl]oxy}ethanol | 13.9 | 11.8 | 10.4 | 6.9 | 5.6 | 2.8 | 0 |
| B | 3,7-Dimethyloct-6-en-1-ol | 8.3 | 7.1 | 6.3 | 4.2 | 3.3 | 1.7 | 0 |
| B | 3a,6,6,9a-tetramethyldodecahydronaphtho[2,1-{b}]furan | 2.8 | 2.4 | 2.1 | 1.4 | 1.1 | 0.6 | 0 |
| A | 1-Methyl-4-(1-methylethyl)-2-[(1E)prop-1-enyl]benzene | 0.0 | 5.4 | 8.9 | 17.9 | 21.4 | 28.6 | 35.7 |
| A | 3-methylcyclopentadecanone | 0.0 | 4.3 | 7.1 | 14.3 | 17.1 | 22.9 | 28.6 |
| A | Ethyl3-(1methylethyl)bicyclo[2.2.1]hept-5-ene-2-carboxylate | 0.0 | 3.2 | 5.4 | 10.7 | 12.9 | 17.1 | 21.4 |
| A | Ylang Ylang oil | 0.0 | 2.1 | 3.6 | 7.1 | 8.6 | 11.4 | 14.3 |
| | **Percent detachment value (PDV)** | **0** | **13** | **34** | **41** | **40** | **36** | **45** |

Table 2 Active perfume composition Athos

| Group | Ingredient | % by weight (total Ingredients 100% $^w/_w$) | | | | | | |
|---|---|---|---|---|---|---|---|---|
| | | 0 | 15 | 25 | 50 | 60 | 80 | 100 |
| B | Rose fragrance 0409™ (available from Quest International) | 11.1 | 9.4 | 8.3 | 5.6 | 4.4 | 2.2 | 0 |
| B | Ethyl-2-methyl-1,3-dioxolan-2-yl acetate | 5.6 | 4.7 | 4.2 | 2.8 | 2.2 | 1.1 | 0 |
| B | 5-heptyldihydrofuran-2(3H)-onc | 2.8 | 2.4 | 2.1 | 1.4 | 1.1 | 0.6 | 0 |
| B | Origanum | 13.9 | 11.8 | 10.4 | 6.9 | 5.6 | 2.8 | 0 |
| B | 1-(methyloxy)propan-2-ol | 13.9 | 11.8 | 10.4 | 6.9 | 5.6 | 2.8 | 0 |
| B | 2,2-dimethyl-3-(3-methylphenyl)propan-1-ol | 8.3 | 7.1 | 6.3 | 42 | 3.3 | 1.7 | 0 |
| B | tncyclo[5.2.1.0^{2,6}]d ec-4-en-8-yl propanoate | 13.9 | 11.8 | 10.4 | 6.9 | 5.6 | 2.8 | 0 |
| B | 1,7,7-Trinethylbicyclo[2.2.1]h ept-2-yl acetate | 5.6 | 4.7 | 4.2 | 2.8 | 2.2 | 1.1 | 0 |
| B | 2-{[2-(Ethyloxy)ethyl]oxy}etha nol | 13.9 | 11.8 | 10.4 | 6.9 | 5.6 | 2.8 | 0 |
| B | 3,7-Dimethyloct-6-en-1-ol | 8.3 | 7.1 | 6.3 | 42 | 3.3 | 1.7 | 0 |
| B | 3a,6,6,9a-tetramethyldodecahydron aphtho[2,1-{b}]furan | 2.8 | 2.4 | 2.1 | 1.4 | 1.1 | 0.6 | 0 |
| A | (2E)-tridec-2-enenitrile | 0.0 | 5.4 | 8.9 | 17.9 | 21.4 | 28.6 | 35.7 |
| A | 1-(2,6,6,8-tetramethyltricydo[5.3.1.0^{1,5}] undec-8-en-9-yl)ethanone | 0.0 | 4.3 | 7.1 | 14.3 | 17.1 | 22.9 | 28.6 |
| A | 1-methylethyl tetradecanoate | 0.0 | 3.2 | 5.4 | 10.7 | 12.9 | 17.1 | 21.4 |
| A | (2Z)-2-ethyl-4-(2,2,3-trimethylcyclopent-3-en-1-yl)but-2-en-1-ol | 0.0 | 2.1 | 3.6 | 7.1 | 8.6 | 11.4 | 14.3 |
| | **Percent detachment value (PDV)** | **0** | **42** | **54** | **68** | **62** | **64** | **57** |

Table 3 Antagonist perfume composition Porthos:

| Group | Ingredient | % by weight (total ingredients 100%$^w$/$_w$) | | | | | | |
|---|---|---|---|---|---|---|---|---|
| | | 0 | 2.5 | 5 | 7.5 | 10 | 15 | 25 |
| B | Rose fragrance 0409™ (available from Quest International) | 11.1 | 10.8 | 10.6 | 10.3 | 10.0 | 9.4 | 8.3 |
| B | Ethyl-2-methyl-1,3-dioxolan-2-yl acetate | 5.6 | 5.4 | 5.3 | 5.1 | 5.0 | 4.7 | 4.2 |
| B | 5-heptyldihydrofuran-2(3H)-one | 2.8 | 2.7 | 2.6 | 2.6 | 2.5 | 2.4 | 2.1 |
| B | Origanum | 13.9 | 13.5 | 13.2 | 12.8 | 12.5 | 11.8 | 10.4 |
| B | 1-(methyloxy)propan-2-ol | 13.9 | 13.5 | 13.2 | 12.8 | 12.5 | 11.8 | 10.4 |
| B | 2,2-dimethyl-3-(3-methylphenyl)propan-1-ol | 8.3 | 8.1 | 7.9 | 7.7 | 7.5 | 7.1 | 6.3 |
| B | tricyclo[5.2.1.0^{2,6}]dec-4-en-8-yl propanoate | 13.9 | 13.5 | 13.2 | 12.8 | 12.5 | 11.8 | 10.4 |
| B | 1,7,7-Trimethylbicyclo[2.2.1]hept-2-yl acetate | 5.6 | 5.4 | 5.3 | 5.1 | 5.0 | 4.7 | 4.2 |
| B | 2-{[2-(Ethyloxy)ethyl]oxy}ethanol | 13.9 | 13.5 | 13.2 | 12.8 | 12.5 | 11.8 | 10.4 |
| B | 3,7-Dimethyloct-6-en-1-ol | 8.3 | 8.1 | 7.9 | 7.7 | 7.5 | 7.1 | 6.3 |
| B | 3a,6,6,9a-tetramethyldodecahydronaphtho[2,1-{b}]furan | 2.8 | 2.7 | 2.6 | 2.6 | 2.5 | 2.4 | 2.1 |
| C | 2-methyl-3-[4-(methyoxy) phenyl] propanal | 0.0 | 0.9 | 1.8 | 2.7 | 3.6 | 5.4 | 8.9 |
| C | (2E)-3-phenylprop-2-enal | 0.0 | 0.7 | 1.4 | 2.1 | 2.9 | 4.3 | 7.1 |
| C | Lemongrass oil | 0.0 | 0.5 | 1.1 | 1.6 | 2.1 | 3.2 | 5.4 |
| C | 1,3-dimethylbut-3-enyl 2-methylpropanoate | 0.0 | 0.4 | 0.7 | 1.1 | 1.4 | 2.1 | 3.6 |
| | Percent detachment value (PDV) | 0 | -30 | -27 | -37 | -40 | -46 | -50 |

Table 3 (continued)

| Group | Ingredient | % by weight (total ingredients 100%$^w/_w$) | | | |
|---|---|---|---|---|---|
| | | 50 | 60 | .80 | 100 |
| B | Rose fragrance 0409™ (available from Quest International) | 5.6 | 4.4 | 2.2 | 0 |
| B | Ethyl-2-methyl-1,3-dioxolan-2-yl acetate | 2.8 | 2.2 | 1.1 | 0 |
| B | 5-heptyldihydrofuran-2(3H)-one | 1.4 | 1.1 | 0.6 | 0 |
| B | Origanum | 6.9 | 5.6 | 2.8 | 0 |
| B | 1-(methyloxy)propan-2-ol | 6.9 | 5.6 | 2.8 | 0 |
| B | 2,2-dimethyl-3-(3-methylphenyl)propan-1-ol | 4.2 | 3.3 | 1.7 | 0 |
| B | tricyclo[5.2.1.0^{2,6}]dec-4-en-8-yl propanoate | 6.9 | 5.6 | 2.8 | 0 |
| B | 1,7,7-Trimethylbicyclo[2.2.1]hept-2-yl acetate | 2.8 | 2.2 | 1.1 | 0 |
| B | 2-{[2-(Ethyloxy)ethyl]oxy}ethanol | 6.9 | 5.6 | 2.8 | 0 |
| B | 3,7-Dimethyloct-6-en-1-ol | 4.2 | 3.3 | 1.7 | 0 |
| B | 3a,6,6,9a-tetramethyldodecahydronaphtho[2,1-{b}]furan | 1.4 | 1.1 | 0.6 | 0 |
| C | 2-methyl-3-[4-(methyoxy) phenyl] propanal | 17.9 | 21.4 | 28.6 | 35.7 |
| C | (2E)-3-phenylprop-2-enal | 14.3 | 17.1 | 22.9 | 28.6 |
| C | Lemongrass oil . | 10.7 | 12.9 | 17.1 | 21.4 |
| C | 1,3-dimethylbut-3-enyl 2-methylpropanoate | 7.1 | 8.6 | 11.4 | 14.3 |
| | Percent detachment value (PDV) | -53 | -53 | -53 | -66 |

16

These results are summarised in Figure 1. A plateau response can be observed at ≥ 25 % w/w active (Group A) ingredients in perfume compositions Aramis and Athos.

For comparison, similar tests were also performed on a typical household cleaning product perfume with the following composition:

Table 4 Typical household cleaning product perfume

| - Ingredient | 100% $^w/_w$ |
|---|---|
| ALDEHYDE C10 (DECANAL) | 5 |
| CAMPHOR POWDER SYNTHETIC (1,7,7-trimethylbicyclo[2.2.1]heptan-2-one) | 02 |
| CINEOLE PQ (1,3,3-trimethyl-2-oxabicyclo[2.2.2] octane) | 2.5 |
| CIS 3 HEXENOL ((3Z)-hex-3-en-1-ol)(10 % solution in DPG) | 0.3 |
| CITRONELLOL PURE (3,7-dimethyloct-6-en-1-ol) | 5 |
| DIHYDRO MYRCENOL (Q) (2,6-dimethylhept-5-en-2-ol) | 19 |
| DIPROPYLENE GLYCOL (1-[(2-hydroxypropyl)ory]propan-2-ol) | 27.5 |
| ETHYL BUTYRATE (10% solution in DPG)(ethyl butanoate) | 0.5 |
| GERANIOL 70 ((2E)-3,7-dimethylocta-2,6-dien-1-ol) | 5 |
| HEXYL CINNAMIC ALDEHYDE ((2E)-2-hexyl-3-phenylprop-2-enal) | 5 |
| ISO BORNYL ACETATE (1,7,7-trimethylbicydo[2.2.1]hept-2-yl acetate) | 11 |
| ISO AMBOIS SUPER CI (Q) (1-(2,3,8,8-tetramethyl-1,2,3,4,5,6,7,8-octahydronaphthalen-2-yl) ethanone) | 0.5 |
| LEMONGRASS oil | 1 |
| LIGUSTRAL (Q) (2,4-dimethylcyclohex-3-ene-1-carbaldehyde) | 1 |
| ETHYL DIHYDRO JASMONATE SUPER (Q) (methyl ((1R,2S)-3-oxo-2-pentyicyclopentyl] acetate) | 4.5 |
| NEROL STD ((2Z)-3,7-dimethylocta-2,6-dien-1-ol) | 3 |
| ORANGE BOOSTER B 5535 (Q) | 1.5 |
| ORANGE BRAZIL PURE | 2 |
| PHENYL ETHYL ALCOHOL (2-phenylethanol) | 0.5 |
| TERPINEOL (2-(4-methylcyclohex-3-en-1-yl)propan-2-ol) | 1 |
| TERPINOLENE EXTRA (1-methyl-4-(1-methylethylidene)cyclohex-1-ene) | 2 |
| TERPINYL ACETATE (1-methyl-1-(4-methylcyclohex-3-en-1-yl)ethyl acetate) | 2 |
| **Percent detachment value (PDV)** | **-43** |
| *DPG = dipropylene glycol  Q indicates material available from Quest International. | |

Example 6

Effect of increasing levels of antagonist perfume in active perfume compositions

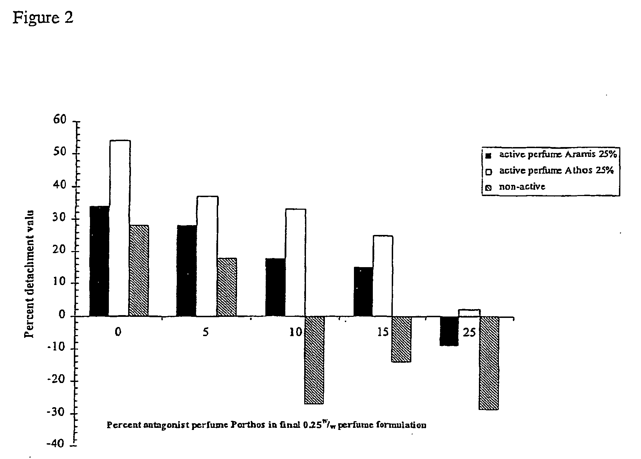

The robustness of biofilm detaching performance of perfume compositions Aramis and Athos of Example 3 (with 25 % of active perfume ingredients) in the presence of increasing amounts of antagonist perfume Porthos (5-25% of final perfume concentration) were investigated. The balance of the perfume formulation consisted of non-active (Group B) ingredients. The final perfume concentration was 0.25 %w/w, prepared in quarter strength GPC base. These data are presented in figure 2 and the perfume formulations are listed in Tables 5, 6 and 7.

Table 5 Perfume composition Aramis (25% of final perfume concentration):

| Group | Ingredient | % by weight (total Ingredients 100%ʷ/w) | | | | |
|---|---|---|---|---|---|---|
| | | 0 | 5 | 10 | 15 | 25 |
| B | Rose fragrance 0409™ (available from Quest International) | 8.3 | 7.8 | 7.2 | 6.7 | 5.6 |
| B | Ethyl-2-methyl-1,3-dioxolan-2-yl acetate | 4.2 | 3.9 | 3.6 | 3.3 | 2.8 |
| B | 5-heptyldihydrofuran-2(3H)-one | 2.1 | 1.9 | 1.8 | 1.7 | 1.4 |
| B | Origanum | 10.4 | 9.7 | 9.0 | 8.3 | 6.9 |
| B | 1-(methyloxy)propan-2-ol | 10.4 | 9.7 | 9.0 | 8.3 | 6.9 |
| B | 2,2-dimethyl-3-(3-methylphenyl)propan-1-ol | 6.3 | 5.8 | 5.4 | 5.0 | 4.2 |
| B | tricyclo[5.2.1.0^{2,6}]dec-4-en-8-yl propanoate | 10.4 | 9.7 | 9.0 | 8.3 | 6.9 |
| B | 1,7,7-Trimethylbicyclo[2.2.1]hept-2-yl acetate | 4.2 | 3.9 | 3.6 | 3.3 | 2.8 |
| B | 2-{[2-(Ethyloxy)ethyl]oxy}ethanol | 10.4 | 9.7 | 9.0 | 8.3 | 6.9 |
| B | 3,7-Dimethyloct-6-en-1-ol | 6.3 | 5.8 | 5.4 | 5.0 | 4.2 |
| B | 3a,6,6,9a-tetramethyldodecahydronaphtho [2,1-{b}]furan | 2.1 | 1.9 | 1.8 | 1.7 | 1.4 |
| A | 1-Methyl-4-(1-methylethyl)-2-[(1E)-prop-1-enyl]benzene | 8.9 | 8.9 | 8.9 | 8.9 | 8.9 |
| A | 3-methylcyclopentadecanone | 7.1 | 7.1 | 7.1 | 7.1 | 7.1 |
| A | ethyl 3-(1-methylethyl)bicyclo[2.2.1]hept-5-ene-2-carboxylate | 5.4 | 5.4 | 5.4 | 5.4 | 5.4 |
| A | Ylang Ylang oil | 3.6 | 3.6 | 3.6 | 3.6 | 3.6 |
| C | 2-methyl-3-[4-(methyoxy) phenyl] propanal | 0.0 | 1.8 | 3.6 | 5.4 | 8.9 |
| C | (2E)-3-phenylprop-2-enal | 0.0 | 1.4 | 2.9 | 4.3 | 7.1 |
| C | lemongrass oil | 0.0 | 1.1 | 2.1 | 3.2 | 5.4 |
| C | 1,3-dimethylbut-3-enyl 2-methylpropanoate | 0.0 | 0.7 | 1.4 | 2.1 | 3.6 |
| | **PDV** | **34** | **28** | **18** | **15** | **-9** |

Table 6 Perfume composition Athos (25% of final perfume concentration):

| Group | Ingredient | % by weight (total Ingredients 100%ʷ/w) | | | | |
|---|---|---|---|---|---|---|
| | | 0 | 5 | 10 | 15 | 25 |
| A | 1-(2,6,6,8-tetramethyltricyclo[5.3.1.0^{1,5}]undec-8-on-9-yl)ethanone | 7.1 | 7.1 | 7.1 | 7.1 | 7.1 |
| A | 1-methylethyl tetradecanoate | 5.4 | 5.4 | 5.4 | 5.4 | 5.4 |
| A | (2Z)-2-ethy4-4-(2,2,3-trimethylcyclopent-3-en-1-yl)but-2-en-1-ol | 3.6 | 3.6 | 3.6 | 3.6 | 3.6 |
| C | 2-methyl-3-[4-(methyoxy) phenyl] propanal | 0.0 | 1.8 | 3.6 | 5.4 | 8.9 |
| C | (ZE)-3-phenylprop-2-enal | 0.0 | 1.4 | 2.9 | 4.3 | 7.1 |
| C | lemongrass oil | 0.0 | 1.1 | 2.1 | 3.2 | 5.4 |
| C | 1,3-dimethylbut-3-enyl 2-methylpropanoate | 0.0 | 0.7 | 1.4 | 2.1 | 3.6 |
| | PDV | 54 | 37 | 33 | 25 | 2 |
| Group | Ingredient | % by weight (total ingredients 100%w/w) | | | | |
| | | 0 | 5 | 10 | 15 | 25 |
| B | Rose fragrance 0409™ (available from Quest International) | 8.3 | 7.8 | 7.2 | 6.7 | 5.6 |

(continued)

| Group | Ingredient | % by weight (total ingredients 100%w/w) | | | | |
|-------|-----------|------|------|------|------|------|
| | | 0 | 5 | 10 | 15 | 25 |
| B | Ethyl-2-methyl-1,3-dioxolan-2-yl acetate | 4.2 | 3.9 | 3.6 | 3.3 | 2.8 |
| B | 5-heptyldihydrofuran-2(3H)-one | 2.1 | 1.9 | 1.8 | 1.7 | 1.4 |
| B | Origanum | 10.4 | 9.7 | 9.0 | 8.3 | 6.9 |
| B | 1-(methyloxy)propan-2-ol | 10.4 | 9.7 | 9.0 | 8.3 | 6.9 |
| B | 2,2-dimethyl-3-(3-methylphenyl)propan-1-ol | 6.3 | 5.8 | 5.4 | 5.0 | 4.2 |
| B | tricyclo[5.2.1.0^{2,6}]dec-4-en-8-yl propanoate | 10.4 | 9.7 | 9.0 | 8.3 | 6.9 |
| B | 1,7,7-Trimethylbicyclo[2.2.1]hept-2-yl acetate | 4.2 | 3.9 | 3.6 | 3.3 | 2.8 |
| B | 2-{[2-(Ethyloxy)ethyl]oxy}ethanol | 10.4 | 9.7 | 9.0 | 8.3 | 6.9 |
| B | 3,7-Dimethyloct-6-en-1-ol | 6.3 | 5.8 | 5.4 | 5.0 | 4.2 |
| B | 3a,6,6,9a-tetramethyldodecahydronaphtho[2,1-{b}]furan | 2.1 | 1.9 | 1.8 | 1.7 | 1.4 |
| A | (2E)-tridec-2-enenitrile | 8.9 | 8.9 | 8.9 | 8.9 | 8.9 |

Table 7 Non-active perfume

| Group | Ingredient | % by weight (total Ingredients 100%w/w) | | | | |
|-------|-----------|------|------|------|------|------|
| | | 0 | 5 | 10 | 15 | 25 |
| B | Rose fragrance 0409™ (available from Quest International) | 11.1 | 10.6 | 10.0 | 9.4 | 8.3 |
| B | Ethyl-2-methyl-1,3-dioxolan-2-yl acetate | 5.6 | 5.3 | 5.0 | 4.7 | 4.2 |
| B | 5-heptyldihydrofuran-2(3H)-one | 2.8 | 2.6 | 2.5 | 2.4 | 2.1 |
| B | Origanum | 13.9 | 13.2 | 12.5 | 11.8 | 10.4 |
| B | 1-(methyloxy)propan-2-ol | 13.9 | 13.2 | 12.5 | 11.8 | 10.4 |
| B | 2,2-dimethyl-3-(3-methylphenyl)propan-1-ol | 8.3 | 7.9 | 7.5 | 7.1 | 6.3 |
| B | tricyclo[5.2.1.0^{2,6}]dec-4-en-8-yl propanoate | 13.9 | 13.2 | 12.5 | 11.8 | 10.4 |
| B | 1,7,7-Trimethylbicyclo[2.2.1]hept-2-yl acetate | 5.6 | 5.3 | 5.0 | 4.7 | 4.2 |
| B | 2-{[2-(Ethyloxy)ethyl]oxy}ethanol | 13.9 | 13.2 | 12.5 | 11.8 | 10.4 |
| B | 3,7-Dimethyloct-6-en-1-ol | 8.3 | 7.9 | 7.5 | 7.1 | 6.3 |
| B | 3a,6,6,9a-tetramethyldodecahydronaphtho[2,1-{b}]furan | 2.8 | 2.6 | 2.5 | 2.4 | 2.1 |
| C | -methyl-3-[4-(methyoxy) phenyl] propanal | 0.0 | 1.8 | 3.6 | 5.4 | 8.9 |
| C | (2E)-3-phenylprop-2-enal | 0.0 | 1.4 | 2.9 | 4.3 | 7.1 |
| C | lemongrass oil | 0.0 | 1.1 | 2.1 | 3.2 | 5.4 |
| C | 1,3-dimethylbut-3-enyl 2-methylpropanoate | 0.0 | 0.7 | 1.4 | 2.1 | 3.6 |
| | **PDV** | **28** | **18** | **-27** | **-14** | **-29** |

These results are summarised in Figure 2.

Example 7

Perfumes prepared in de-ionised water

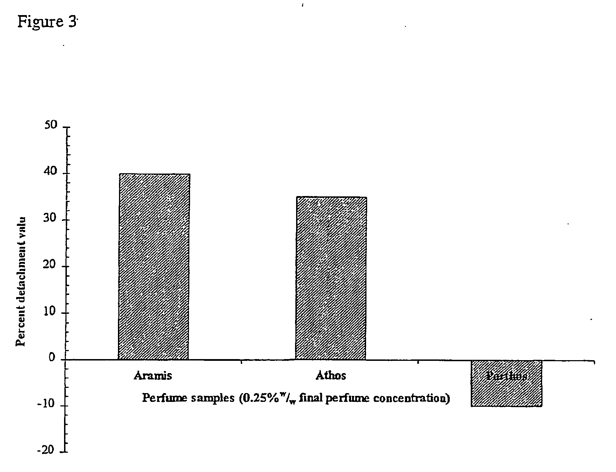

15 component perfume formulations (including 11 non-active (Group B) ingredients) containing either four active (Group A) or 4 antagonist (Group C) ingredients (at 50% of final perfume concentration) were prepared in de-ionised water. The final perfume concentration was 0.25 %w/w. The perfume formulations are listed (Table 8) together with percent detachment values. Results are shown in Figure 3.

Table 8 Perfume formulations prepared in de-ionised water

| Group | Ingredients | Perfume formulations 100%$^w/_w$ | | |
|---|---|---|---|---|
| | | Aramis | Athos | Porthos |
| B | Rose fragrance 0409T™ (available from Quest International) | 5.6 | 5.6 | 5.6 |
| B | Ethyl-2-methyl-1,3-dioxolan-2-yl acetate | 2.8 | 2.8 | 2.8 |
| B | 5-heptyldihydrofuran-2(3H)-one | 1.4 | 1.4 | 1.4. |
| B | Origanum | 6.9 | 6.9 | 6.9 |
| B | 1-(methyloxy)propan-2-ol | 6.9 | 6.9 | 6.9 |
| B | 2,2-dimethyl-3-(3-methylphenyl)propan-1-ol | 4.2 | 42 | 4.2 |
| B | tricyclo[5.2.1.0^{2,6}]dec-4-en-8-yl propanoate | 6.9 | 6.9 | 6.9 |
| B | 1,7,7-Trimethylbicyclo[2.2.1]hept-2-yl acetate | 2.8 | 2.8 | 2.8 |
| B | 2-{[2-(Ethyloxy)ethyl]oxy}ethanol | 6.9 | 6.9 | 6.9 |
| B | 3,7-Dimethyloct-6-en-1-ol | 4.2 | 42 | 4.2 |
| B | 3a,6,6,9a-tetramethyldodecahydronaphtho[2,1-{b}]furan | 1.4 | 1.4 | 1.4 |
| A | 1-Methyl-4-(1-methylethyl)-2-[(1E)-prop-1-enyl]benzene | 17.9 | 0 | 0 |
| A | 3-methylcyclopentadecanone | 14.3 | 0 | 0 |
| A | Ethyl 3-(1methylethyl)bicyclo[2.2.1] hept-5-ene-2-carboxylate | 10.7 | 0 | 0 |
| A | Ylang Ylang oil | 7.1 | 0 | 0 |
| A | (2E)-tridec-2-enenitrile | 0 | 17.9 | 0 |
| A | 1-(2,6,6,8-tetramethyltricyclo[5.3.1.0^{1,5}]undec-8-en-9-yl) ethanone | 0 | 14.3 | 0 |
| A | 1-methylethyl tetradecanoate | 0 | 10.7 | 0 |
| A | (2Z)-2-ethyl-4-(2,2,3-trimethylcyclopent-3-en-1-yl)but-2-en-1-ol | 0 | 7.1 | 0 |
| C | 2-methyt-3-[4-(methyoxy) phenyl] propanal | 0 | 0 | 17.9 |
| C | (2E)-3-phenylprop-2-enal | 0 | 0 | 14.3 |
| C | Lemongrass oil | 0 | 0 | 10.7 |
| C | 1,3-dimethylbut-3-enyl 2-methylpropanoate | 0 | 0 | 7.1 |
| | **Percent detachment value (PDV)** | **40** | **35** | **-10** |

These results are shown in Figure 3.

**Claims**

1. A perfume composition comprising at least 15% by weight of at least three Group A perfume ingredients and at least one Group B ingredient, and wherein Group A and Group B ingredients together constitute at least 80% by weight of the perfume composition, wherein the Group A ingredients are selected from the group consisting of

(2E)-tridec-2-enenitrile
(2Z)-2-ethyi-4-(2,2,3-trimethylcyclopent-3-en-1-yl)but-2-en-1-ol
(3E)-3-methyl-4-(2,6,6-trimethylcyclohex-2-en-1-yl)but-3-en-2-one
1-(2,3,8,8-tetramethyl-1,2,3,5,6,7,8,8a-octahydronaphthalen-2-yl)ethanone
1-(2,6,6,8-tetramethyl-tricyclo [5.3.1.0^ {1,5}]undec-8∼en-9-yl)ethanone

1-(methyloxy)-4-[(1E)-prop-1-enyl]benzene

1,4-dioxacycloheptadecane-5,17-dione

1-[1,1,2,6-tetramethyl-3-(1-methylethyl)-2,3-dihydro-1H-inden-5-yl]ethanone

1-methyl-4-(1-methylethyl)-2-[(1 E)-prop-1-enyl]benzene

1-methylethyl tetradecanoate

2-(2-methylpropyl)-4-hydroxy-4-methyl-tetrahydropyran

2,6,10-trimethylundec-9-enal

2- [2-(4-methylcyclohex-3-en-1-yl)propyl] cyclopentanone

2-methylundecanal

5-methyl-2-(1-methylethyl)phenol

cinnamic alcohol

clove bud rectified extra DQ P353

cyclohexadecanolide

cyclopentadecanone

decanol DQ

dodecanenitrile

eucalyptol

eucalyptus globulus

eugenol rectified

ginger oils

isoamyl acetate

nonanol

patchouli oil

phenylmethanol

propane-1,2-diol

tea tree oil DQ

tributyl-2-(acetyloxy)propane-1,2,3-tricarboxylate

1 H-indole

(2E)-2-pentyl-3-phenylprop-2-enal

(4E)-4-tricyclo[5.2.1.0^{2,6}]dec-8-ylidenebutanal

2,6,6,8-tetramethyltricyclo[5.3.1.0^{1,5}]undec-8-yl acetate

2-[(2-{[2-(methyloxy)propyl]oxy}propyl)oxy]propan-1-ol

2~methyldecanenitrile

1-(1,1,2,3,3,6-hexamethyl-2,3-dihydro-1H-inden-5-yl)ethanone

1-(3,5,5,6,8,8-hexamethyl-5,6,7,8-tetrahydronaphthalen-2-yl)ethanone

4-(1,7,7-trimethylbicyclo[2.2.1]hept-2-yl)cyclohexanol

Silvanone (Silvanone is a Trade Mark)

2-heptylcyclopentanone

3 -methyldodecanenitrile

(6E)-3,7,11 -trimethyldodeca-1 ,6,10-trien-3-ol

methyl (2E)-non-2-enoate

(2E)-2-hexyl-3-phenylprop-2-enal

ethyl 3-(1-methylethyl)bicyclo[2.2.1]hept-5-ene-2-carboxylate

3-methylcyclopentadecanone

ylang ylang oil

2-(1,1-dimethylethyl)cyclohexyl acetate.

2,4-dimethyl-4,4a,5,9b-tetrahydroindeno[1,2-{d}][1,3]dioxine

4,6,6,7,8,8-hexamethyl-1,3,4,6,7,8-hexahydroindeno[5,6-{c}]pyran (e.g. in the form of Galaxolide - Galaxolide is .a Trade Mark)

[4-(1-methylethyl)cyclohexyl]methanol

capsicum oleoresin DQ

lime terpeneless DQ

citronella Ceylon DQ

rose flavour base ABF0339A

marjoram French DQ

jasmin absolute DQ

coriander DQ

lavender oil DQ

iso propyl alcohol DQ
amyl cinnamic aldehyde DQ
amyl caproate DQ

ketones of general formula RCOR' having an octanol-water partition coefficient of at least 4 (expressed as. a logarithm to base 10), where R and R' are independently hydrocarbyl residues that may be aliphatic or aromatic, saturated or unsaturated, and combinations thereof, but may not contain, other functional groups, including

1-(2,3,8,8-tetramethyl-1,2,3,4,5,6,7,8-octahydronaphthalen-2-yl)ethanone (log P = 5.28)
1-(2,6,6,8-tetramethyltricyclo[5.3.1.0^{1,5}undec-8-en-9-yl)ethanone (log P = 5.17)
1-(1,1,2,3,3,6-hexamethyl-2,3-dihydro-1H-inden-5-yl)ethanone (log P = 5.80)
1-(3,5,5,6,8,8-hexamethyl-5,6,7,8-tetrahydronaphthalen-2-yl)ethanone (log P = 6.37)
3-methylcyclopentadecanone (log P = 6.33)
1-[1,1,2,6-tetramethyl-3-(1-methylethyl)-2,3-dihydro-1H-inden-5-yl]ethanone (log P = 6.14)

and Group B ingredients are selected from the group consisting of

(3Z)-hex-3-en-1-ol
1,7,7-trimethylbicyclo[2.2.1]hept-2-yl acetate
1-methyl-4-(1-methylethylidene)cyclohex-1-ene
2-(phenyloxy)ethanol
2,6,10-trimethyl-1-acetyl-cyclododeca-2,5,9-triene
2-{[2-(ethyloxy)ethyl]oxy}ethanol
3,7-dimethyloct-6-en-1-ol
diethyl benzene-1,2-dicarboxylate
(4E)-dec-4-enal
ethyl-2-methyl-I ,3-dioxolan-2-yl acetate
5-hepxyldihydrofuran-2(3H)-one
origanum
1 -(methyloxy)propan-2-ol
2,2-dimethyl-3-(3-methylphenyl)propan-1-ol
tricyclo[5.2.1.0^{2,6}]dec-4-en-8-yl propanoate
3a,6,6,9a-tetramethyldodecahydronaphthol [2,1-{b}]furan
hexyl 2-hydroxybenzoate
phenylmethyl-2-hydroxybenzoate
cyclohexadec-5-en-1-one
Rose fragrance 0409(TM) (available from Quest International)
1-ethenyl-1 ,5-dimethylhex-4-enyl acetate
(4Z)-dec-4-enal
1-(5,5-dimethylcyclohex-1-en-1-yl)pent-4-en-1-one
3-methyl-1-(2-methylpropyl)butyl acetate
2-(methyloxy)-4-propylphenol
2-(acetyloxy)-1-[(acetyloxy)methyl] ethyl acetate
Thyme Red
4-(1,1 -dimethylethyl)cyclohexyl acetate
5-methyl-2-(I-methylethyl)cyclohexanol
Aldehyde C10 DQ
Lime Terpenes Washed DQ
Blackcurrant Base ABF0972
Grapefruit DQ
Parsley Herb
Orange Terpenes Ex Concentrate DO
Iso Amyl Butyrate DQ
Cinnamon Supra ABF1092
Cardamom English Distilled DQ
Apple Base ABF1016
Orange Terpeneless DQ

22

2. A perfume composition according to claim 1, comprising at least 25% by weight, preferably at least 30% by weight, preferably at least 40% or possibly at least 60% by weight of Group A perfume ingredients.

3. A perfume composition according to any one preceding claim, comprising at least 3 Group B perfume ingredients.

4. A perfume composition according to any one preceding claim, which comprises at most 15% of Group C ingredients wherein the Group C ingredients are selected from the group consisting of:

> 2-methyl-3-[4-(methyoxy) phenyl] propanal
> lemongrass oil
> 1,3-dimethylbut-3-enyl 2-methylpropanoate
> Muguet base AB7001 (TM) (Q)
> Moss base AB7004(TM) (Q)
> Sandalone AC802(TM) (Q)
> Jasmin AB7002(TM) (Q)
> prop-2-enyl [(2-methylbutyl)oxy] acetate
> 2,6-dimethylhept-5-en-2-ol
> phenylmethyl acetate
> Carvone
> 3-pentyltetrahydro-2H-pyran-4-yl acetate
> 3 ,7-dimethylocta-1,6-dien-3-ol
> 4-(4-hydroxy-4-methylpentyl)cyclohex-3-ene-1-carbaldehyde
> methyl-(3-oxo-2-pentylcyclopentyl)acetate
> 3-methyl-5-phenylpentan-1-ol
> (1 E)-1-(2,6,6-trimethylcyclohex-2-en-1-yl)pent-1-en-3-one
> Orange oil
> 2-phenylethanol
> 2-(4-methylcyclohex-3-en-1-yl)propan-2-ol
> 1-[(2-hydroxypropyl)oxy]propan-2-ol
> (6Z)-3,7-dimethylnona-1,6-dien-3-ol

5. A perfume composition according to any one of the preceding claims, wherein each perfume ingredient is present in an amount not exceeding 20% by weight.

6. A perfume composition according to any one of the preceding claims, wherein at least one of the perfume ingredients has antimicrobial properties.

7. A perfumed product comprising a perfume composition in accordance with any one of the preceding claims wherein the perfumed product is a household cleaning product, a dental care product or a deodorant product.

8. A method of detaching a biofilm from a surface, comprising application to the surface of a perfume composition or perfumed product in accordance with any one of claims 1 to 7 wherein the surface is a household surface, an oral surface or a human skin surface.

9. Use of a perfume composition as defined in claim 1 for the purpose of detaching a biofilm.


**Patentansprüche**

1. Parfümzusammensetzung, umfassend mindestens 15 Ges.-% mindestens drei Parfümbestandteile der Gruppe A und mindestens einen Bestandteil der Gruppe B, wobei die Bestandteile der Gruppe A und Gruppe B zusammen mindestens 80 Ges.-% der Parfümzusammensetzung ausmachen, wobei die Bestandteile der Gruppe A aus der Gruppe bestehend aus (2E)-Tridec-2-ennitril, (2Z)-2-Ethyl-4-(2,2,3-trimethylcyclopent-3-en-1-yl)but-2-en-1-ol, (3E)-3-Methyl-4-(2,6,6-trimethylcyclohex-2-en-1-yl)but3-en-2-on, 1-(2,3,8,8-Tetramethyl-1,2,3,5,6,7,8,8a-octahydronaphthalin-2-yl)ethanon, 1-(2,6,6,8-Tetramethyltricyclo[5.3.1.0^{1,5}]undec-8-en-9-yl)ethanon, 1-(Methyloxy)-4-[(1E)-prop-1-enyl]benzol, 1,4-Dioxacycloheptadecan-5,17-dion, 1-[1,1,2,6-Tetramethyl-3-(1-methylethyl)-2,3-di-hydro-1H-inden-5-yl]-ethanon, 1-Methyl-4(1-methylethyl)-2-[(1E)-prop-1-enyl]benzol, Tetradecansäure-1-methylethylester, 2-(2-Methylpropyl)-4-hydroxy-4-methyltetrahydropyran, 2,6,10-Trimethylundec-9-enal, 2-[2-(4-Methylcyclohex-3-en-1-yl)propyl]cyclopentanon, 2-Methylundecanal, 5-Methyl-2-(1-methylethyl)phenol, Zimtalkohol, rektifiziertes Nelkenknospenöl Extra DQ P353, Cyclohexadecanolid, Cyclopentadecanon, Decanol DQ, Dodecanitril, Eukalyptol, Eucalyptus globulus, rektifiziertes Eugenol, Ingweröle, Essigsäureisoamylester, Patchouliöl, Phenylmethanol, Propan-1,2-diol, Teebaumöl DQ, Tributyl-2-(acetyloxy)propan-1,2,3-tricarboxylat, 1H-Indol, (2E)-2-Pentyl-3-phenylprop-2-enal, (4E)-4-Tricyclo[5.2.1.0^{2,6}]dec-8-ylidenbutanal, Essigsäure-2,6,6,8-tetramethyltricyclo[5.3.1.0^{1,5}]undec-8-ylester, 2-[(2-{[2-(Methyloxy)propyl]oxy}propyl)oxy]propan-1-ol, 2-Methyldecannitril, 1-(1,1,2,3,3,6-Hexamethyl-2,3-dihydro-1H-inden-5-yl)ethanon, 1-(3,5,5,6,8,8-Hexamethyl-5,6,7,8-tetrahydronaphthalin-2-yl)-ethanon, 4-(1,7,7-Trimethylbicyclo[2.2.1]hept-2-yl)cyclohexanol, Silvanone (Silvanone ist ein Warenzeichen), 2-Heptylcyclopentanon, 3-Methyldodecannitril, (6E)-3,7,11-Trimethyldodeca-1,6,10-trien-3-ol, (2E)-Non-2-ensäuremethylester, (2E)-2-Hexyl-3-phenylprop-2-enal, 3-(1-Methylethyl)-bicyclo[2.2.1]hept-5-en-2-carbonsäureethylester, 3-Methylcyclopentadecanon, Ylang-Ylang-Öl, Essigsäure-2-(1,1-dimethylethyl)cyclohexylester, 2,4-Dimethyl-4,4a,5,9b-tetrahydroindeno[1,2-{d}][1,3]-dioxin, 4,6,6,7,8,8-Hexamethyl-1,3,4,6,7,8-hexahydroindeno[5,6-{c}]pyran (z.B. in Form von Galaxolide - Galaxolide ist ein Warenzeichen), [4-(1-Methylethyl)cyclohexyl]methanol, Capsicum-Oleoresin DQ, Limone terpenfrei DQ, Citronella Ceylon DQ, Rosengeschmacksbasis ABF0339A, Französischer Majoran DQ, Jasmin-Absolue DQ, Koriander DQ, Lavendelöl DQ, Isopropylalkohol DQ, Amylzimtaldehyd DQ, Caprinsäureamylester DQ und Ketonen der allgemeinen Formel RCOR' mit einem Octanol-Wasser-Verteilungskoeffizienten von mindestens 4 (ausgedrückt als Logarithmus zur Basis 10), wobei R und R' unabhängig voneinander für Hydrocarbylreste, die aliphatisch oder aromatisch, gesättigt oder ungesättigt sein können, und Kombinationen davon stehen, aber keine weiteren funktionellen Gruppen enthalten dürfen, einschließlich 1-(2,3,8,8-Tetramethyl-1,2,3,4,5,6,7,8-octahydronaphthalin-2-yl)ethanon, 1-(2,6,6,8-Tetramethyltricyclo[5.3.1.0^{1,5}]-undec-8-en-9-yl)ethanon, 1-(1,1,2,3,3,6-Hexamethyl-2,3-dihydro-1H-inden-5-yl)ethanon, 1-(3,5,5,6,8,8-Hexamethyl-5,6,7,8-tetrahydronaphthalin-2-yl)ethanon, 3-Methylcyclopentadecanon, 1-[1,1,2,6-Tetramethyl-3-(1-methylethyl)-2,3-di-hydro-1H-inden-5-yl]ethanon ausgewählt sind und die Bestandteile der Gruppe B aus der Gruppe bestehend aus (3Z)-Hex-3-en-1-ol, Essigsäure-1,7,7-trimethylbicyclo[2.2.1]hept-2-ylester, 1-Methyl-4-(1-methylethyliden)cyclohex-1-en, 2-(Phenyloxy)ethanol, 2,6,10-Trimethyl-1-acetyl-cyclododeca-2,5,9-trien, 2-{[2-(Ethyloxy)ethyl]-oxy}ethanol, 3,7-Dimethyloct-6-en-1-ol, Benzol-1,2-dicarbonsäurediethylester, (4E)-Dec-4-enal, Essigsäureethyl-2-methyl-1,3-dioxolan-2-ylester, 5-Heptyldihydrofuran-2(3H)-on, Origanum, 1-(Methyloxy)propan-2-ol, 2,2-Dimethyl-3-(3-methyl-phenyl)propan-1-ol, Propansäuretricyclo-[5.2.1.0^{2,6}]dec-4-en-8-ylester, 3a,6,6,9a-Tetramethyldodecahydronaphtho[2,1-{b}]furan, 2-Hydroxybenzoesäurehexylester, Phenylmethyl-2-hydroxybenzoat, Cyclohexadec-5-en-1-on, Rose Fragrance 0409™ (erhältlich von Quest International), Essigsäure-1-ethenyl-1,5-dimethylhex-4-enylester, (4Z)-Dec-4-enal, 1-(5,5-Dimethylcyclohex-1-en-1-yl)pent-4-en-1-on, Essigsäure-3-methyl-1(2-methylpropyl)butylester, 2-(Methyloxy)-4-propylphenol, Essigsäure-2-(acetyloxy)-1-[(acetyloxy)methyl]ethylester, Thyme Red, Essigsäure-4-(1,1-dimethylethyl)cyclohexylester, 5-Methyl-2-(1-methylethyl)-cyclohexanol, Aldehyde C10 DQ, Lime Terpenes Washed DQ, Blackcurrent Base ABF0972, Grapefruit DQ, Parsley Herb, Orange Terpenes Ex Concentrate DQ, Iso Amyl Butyrate DQ, Cinnamon Supra ABF1092, Cardamom English Distilled DQ, Apple Base ABF1016, Orange Terpeneless DQ ausgewählt sind.

2. Parfümzusammensetzung gemäß Anspruch 1, umfassend mindestens 25 Gew.-%, vorzugsweise mindestens 30 Gew.-%, vorzugsweise mindestens 40 Gew.-% oder eventuell mindestens 60 Gew.-% Parfümbestandteile der Gruppe A.

3. Parfümzusammensetzung nach einem der vorhergehenden Ansprüche, umfassend mindestens 3 Parfümbestandteile der Gruppe B.

4. Parfümzusammensetzung nach einem der vorhergehenden Ansprüche, die höchstens 15 Gew.-% Bestandteile der Gruppe C umfasst, wobei die Bestandteile der Gruppe C aus der Gruppe bestehend aus 2-Methyl-3-[4-(methoxy)phenyl]propanal, Lemongrasöl, 2-Methylpropansäure-1,3-dimethylbut-3-enylester, Muguet-Basis AB 7001™, Moss-Basis AB7004™, Sandalone AC802™, Jasmin AB7002™, [(2-Methylbutyl)oxy]essigsäureprop-2-enylester, 2,6-Dimethylhept-5-en-2-ol, Essigsäurephenylmethylester, Carvone, Essigsäure-3-pentyltetrahydro-2H-pyran-4-ylester, 3,7-Dimethylocta-1,6-dien-3-ol, 4-(4-Hydroxy-4-methylpentyl)cyclohex-3-en-1-carbaldehyd, (3-Oxo-2-pentylcyclopentyl)essigsäuremethylester, 3-Methyl-5-phenylpentan-1-ol, (1E)-1-(2,6,6-Trimethylcyclohex-2-en-1-yl)pent-1-en-3-on, Orangenöl, 2-Phenylethanol, 2-(4-Methylcyclohex-3-en-1-yl)propan-2-ol, 1-[(2-Hydroxypropyl)oxy]propan-2-ol, (6Z)-3,7-Dimethylnona-1,6-dien-3-ol ausgewählt sind.

5. Parfümzusammensetzung nach einem der vorhergehenden Ansprüche, wobei jeder Parfümbestandteil in einer Menge von höchstens 20 Gew.-% vorliegt.

6. Parfümzusammensetzung nach einem der vorhergehenden Ansprüche, wobei mindestens einer der Parfümbestandteile antimikrobielle Eigenschaften aufweist.

7. Parfümiertes Produkt, umfassend eine Parfümzusammensetzung nach einem der vorhergehenden Ansprüche, wobei es sich bei dem parfümierten Produkt um ein Haushaltsreinigungsprodukt, ein Zahnpflegeprodukt oder ein Deoprodukt handelt.

8. Verfahren zum Ablösen eines Biofilms von einer Oberfläche, bei dem man auf die Oberfläche eine Parfümzusammensetzung bzw. ein parfümiertes Produkt nach einem der Ansprüche 1 bis 7 aufbringt, wobei es sich bei der Oberfläche um eine Haushaltsoberfläche, eine orale Oberfläche oder eine Oberfläche menschlicher Haut handelt.

9. Verwendung einer Parfümzusammensetzung gemäß Anspruch 1 zum Ablösen eines Biofilms.

**Revendications**

1. Composition de parfum comprenant au moins 15% en poids d'au moins trois ingrédients de parfum du Groupe A et au moins un ingrédient du Groupe B, et où les ingrédients du Groupe A et du Groupe B constituent ensemble au moins 80% en poids de la composition de parfum, où les ingrédients du Groupe A sont choisis dans le groupe constitué par le (2E)-tridéc-2-ènenitrile, le (2Z)-2-éthyl-4-(2,2,3-triméthyl-cyclopent-3-én-1-yl)but-2-én-1-ol, la (3E)-3-méthyl-4-(2,6,6-triméthylcyclohex-2-én-1-yl)but-3-én-2-one, la 1-(2,3,8,8-tétraméthyl-1,2,3,5,6,7,8,8a-octahydro-naphtalén-2-yl)éthanone, la 1-(2,6,6,8-tétraméthyl-tricyclo[5.3.1.0^{1,5}]undéc-8-én-9-yl)éthanone, le 1-(méthyloxy)-4-[(1E)-prop-1-ényl]benzène, la 1,4-dioxacycloheptadécane-5,17-dione, la 1-[1,1,2,6-tétraméthyl-3-(1-méthyléthyl)-2,3-dihydro-1H-indén-5-yl]éthanone, le 1-méthyl-4-(1-méthyléthyl)-2-[(1E)-prop-1-ényl]benzène, le tétradécanoate de 1-méthyléthyle, le 2-(2-méthylpropyl)-4-hydroxy-4-méthyl-tétrahydropyranne, le 2,6,10-triméthylundéc-9-énal, la 2-[2-(4-méthylcyclohex-3-én-1-yl)propyl]cyclopentanone, le 2-méthylundécanal, le 5-méthyl-2-(1-méthyléthyl)-phénol, l'alcool cinnamique, la fleur de girofle rectifiée extra DQ (« <u>D</u>ental <u>Q</u>uality [Qualité Dentaire] » P353, le cyclohexadécanolide, la <u>c</u>yclopentadécanone, le décanol DQ, le dodécanenitrile, l'eucalyptol, *Eucalyptus globulus,* l'eugénol rectifié, les essences de gingembre, l'acétate d'isoamyle, l'essence de patchouli, le phénylméthanol, le propane-1,2-diol, l'huile de Mélaleuca à feuilles alternes DQ, le 2-(acétyloxy)propane-1,2,3-tricarboxylate de tributyle, le 1H-indole, le (2E)-2-pentyl-3-phénylprop-2-énal, le (4E)-4-tricyclo[5.2.1.0^{2,6}]déc-8-ylidène-butanal, l'acétate de 2,6,6,8-tétraméthyltricyclo-[5.3.1.0^{1,5}]undéc-8-yle, le 2-[(2-{[2-(méthyloxy)-propyl]oxy}propyl)oxy] propan-1-ol, le 2-méthyl-décanenitrile, la 1-(1,1,2,3,3,6-hexaméthyl-2,3-dihydro-1H-indén-5-yl)éthanone, la 1-(3,5,5,6,8,8-hexaméthyl-5,6,7,8-tétrahydronaphtalén-2-yl)éthanone, le 4-(1,7,7-triméthylbicyclo[2.2.1]hept-2-yl)-cyclohexanol, la Silvanone (Silvanone est une marque de fabrique), la 2-heptylcyclopentanone, le 3-méthyldodécanenitrile, le (6E)-3,7,11-triméthyldodéca-1,6,10-trién-3-ol, le (2E)-non-2-énoate de méthyle, le (2E)-2-hexyl-3-phénylprop-2-énal, le 3-(1-méthyl-éthyl)bicyclo[2.2.1]hept-5-ène-2-carboxylate d'éthyle, la 3-méthylcyclopentadécanone, l'essence d'ylang-ylang, l'acétate de 2-(1,1-diméthyléthyl)cyclohexyle, la 2,4-diméthyl-4,4-a,5,9b-tétrahydroindéno[1,2-{d}][1,3]-dioxine, le 4,6,6,7,8,8-hexaméthyl-1,3,4,6,7,8-hexahydroindéno[5,6-{c}]pyranne (par exemple sous la forme de Galaxolide - Galaxolide est une marque de fabrique), le [4-(1-méthyléthyl)cyclohexyl]méthanol, l'oléorésine de poivron DQ, le citron vert sans terpènes DQ, la citronnelle de Ceylan DQ, la base d'arôme de rose ABF0339A, la marjolaine française DQ, le jasmin absolu DQ, le coriandre DQ, l'essence de lavande DQ, l'alcool isopropylique DQ, l'aldéhyde amylcinnamique DQ, le caproate d'amyle DQ, et les cétones de formule générale RCOR' ayant un coefficient de partage dans l'octanol-eau d'au moins 4 (exprimé comme logarithme en base 10), où R et R' sont indépendamment des restes hydrocarbyle pouvant être aliphatiques ou aromatiques, saturés ou insaturés, et des combinaisons de ceux-ci, mais ne peuvent pas contenir d'autres groupements fonctionnels, y compris la 1-(2,3,8,8-tétraméthyl-1,2,3,4,5,6,7,8-octahydronaphtalén-2-yl)éthanone, la 1-(2,6,6,8-tétraméthyltricyclo[5.3.1.0^{1,5}]undéc-8-én-9-yl)éthanone, la 1-(1,1,2,3,3,6-hexaméthyl-2,3-dihydro-1H-indén-5-yl)éthanone, la 1-(3,5,5,6,8,8-hexaméthyl-5,6,7,8-tétrahydronaphtalén-2-yl)éthanone, la 3-méthylcyclopentadécanone, la 1-[1,1,2,6-tétraméthyl-3-(1-méthyléthyl)-2,3-dihydro-1H-indén-5-yl]éthanone ;
et les ingrédients du Groupe B sont choisi dans le groupe constitué par l'acétate de (3Z)-hex-3-én-1-ol, 1,7,7-triméthylbicyclo[2.2.1]hept-2-yle, le 1-méthyl-4-(1-méthyléthylidène)cyclohex-1-ène, le 2-(phényloxy)éthanol, le 2,6,10-triméthyl-1-acétyl-cyclododéca-2,5,9-triène, le 2-{[2-(éthyloxy)éthyl]-oxy}éthanol, le 3,7-diméthyloct-6-én-1-ol, le benzène-1,2-dicarboxylate de diéthyle, le (4E)-déc-4-énal, l'acétate d'éthyl-2-méthyl-1,3-dioxolan-2-yle, la 5-heptyldihydrofuran-2(3H)-one, *Origanum,* le 1-(méthyloxy)propan-2-ol, le 2,2-diméthyl-3-(3-méthylphényl)propan-1-ol, le propanoate de tricyclo[5.2.1.0^{2,6}]déc-4-én-8-yle, le 3a,6,6,9a-tétraméthyldodécahydronaphto[2,1-{b}]furane, le 2-hydroxybenzoate d'hexyle, le 2-hydroxybenzoate de phénylméthyle, la cyclohexadéc-5-én-1-one, le parfum de Rose 0409™ (disponible auprès de Quest International), l'acétate de 1-ethényl-1,5-diméthylhex-4-ényle, le (4Z)-déc-4-énal, la 1-(5,5-diméthylcyclohex-1-én-1-yl)pent-4-én-1-one, l'acétate de 3-méthyl-1-(2-méthylpropyl)butyle, le 2-(méthyloxy)-4-propylphénol, l'acétate de 2-(acétyloxy)-1-[(acétyloxy)méthyl]éthyle, le Thym rouge, l'acétate de 4-(1,1-diméthyléthyl)-cyclohexyle, le 5-méthyl-2-(1-méthyléthyl)cyclohexanol, l'Aldéhyde C10 DQ, les terpènes de citron vert lavés DQ, la base de cassis ABF0972, le pamplemousse DQ, le persil, le concentré EX de terpènes d'orange DQ, le butyrate d'isoamyle DQ, la cannelle supra ABF1092, le distillat de Cardamome Anglais DQ, la Base de pomme ABF1016, l'Orange sans terpènes DQ.

2. Composition de parfum selon le revendication 1, comprenant au moins 25% en poids, préférablement au moins 30% en poids, préférablement au moins 40% en poids ou éventuellement au moins 60% en poids d'ingrédients de parfum du Groupe A.

3. Composition de parfum selon l'une quelconque des revendications précédentes, comprenant au moins 3 ingrédients de parfum du Groupe B.

4. Composition de parfum selon l'une quelconque des revendications précédentes, qui comprend au plus 15% en poids d'ingrédients du Groupe C, où les ingrédients du Groupe C sont choisis dans le groupe constitué par le 2-méthyl-3-[4-(méthoxy)phényl]propanal, l'essence de lemon-grass, le 2-méthylpropanoate de 1,3-diméthylbut-3-ényle, la base de Muguet AB7001™, la base de Mousse AB7004™, le Sandalone AC802™, le Jasmin AB7002™, le [(2-méthylbutyl)oxy]acétate de prop-2-ényle, le 2,6-diméthylhept-5-én-2-ol, l'acétate de phénylméthyle, la Carvone, l'acétate de 3-pentyltétrahydro-2H-pyran-4-yle, le 3,7-diméthylocta-1,6-dién-3-ol, le 4-(4-hydroxy-4-méthylpentyl)cyclohex-3-ène-1-carbaldéhyde, le (3-oxo-2-pentylcyclopentyl)acétate de méthyle, le 3-méthyl-5-phénylpentan-1-ol, la (1E)-1-(2,6,6-triméthylcyclohex-2-én-1-yl)pent-1-én-3-one, l'essence d'Orange, le 2-phényléthanol, le 2-(4-méthylcyclohex-3-én-1-yl)propan-2-ol, le 1-[(2-hydroxypropyl)oxy]propan-2-ol, le (6Z)-3,7-diméthylnona-1,6-dién-3-ol.

5. Composition de parfum selon l'une quelconque des revendications précédentes, dans laquelle chaque ingrédient de parfum est présent selon une quantité ne dépassant pas 20% en poids.

6. Composition de parfum selon l'une quelconque des revendications précédentes, dans laquelle au moins l'un des ingrédients de parfum possède des propriétés antimicrobiennes.

7. Produit parfumé comprenant une composition de parfum selon l'une quelconque des revendications précédentes, dans laquelle le produit parfumé est un produit de nettoyage ménager, un produit de soin dentaire ou un produit déodorant.

8. Méthode de décollage d'un biofilm à partir d'une surface, comprenant l'application à la surface d'une composition de parfum ou d'un produit parfumé selon l'une quelconque des revendications 1 à 7, dans laquelle la surface est une surface domestique, une surface orale ou une surface de la peau humaine.

9. Utilisation d'une composition de parfum selon la revendication 1, aux fins de décollage d'un biofilm.

Figure 1

Figure 2

Figure 3

## REFERENCES CITED IN THE DESCRIPTION

### Patent documents cited in the description

- US 6585961 B1
- WO 0124769 A
- US 4289641 A

### Non-patent literature cited in the description

- **S. ARCTANDER.** *Perfume and Flavor Chemicals,* 1969
- **S. ARCTANDER.** *Perfume and Flavor Materials of Natural Origin,* 1960
- Flavor and Fragrance Materials. Allured Publishing Co, 1991
- **COSTERTON JW ; LEWANDOWSKI Z ; CALDWELL DE ; KORBER DR ; LAPPIN- SCOTT HM.** Microbial biofilms. *Annual Reviews of Microbiology,* 1995, vol. 49, 711-745
- **GILBERT P ; MAIRA-LITRAN T ; MCBAIN AJ ; RICKARD AH ; WHYTE FW.** The physiology and collective recalcitrance of microbial biofilm communities. *Advances in Microbial Physiology,* 2002, vol. 46, 202-256
- **HANSEN ; LEO.** *Chemical Reviews,* 1971, vol. 526 to 616, 71
- **HANSCH ; QUINLAN ; LAWRENCE.** *J. Organic Chemistry,* 1968, vol. 347 to 350, 33
- **A LEO.** Calculating log P oct from structures. *Chem. Rev,* 1993, vol. 93 (4), 1281-1306
- **MORRIS J A ; KHETTRY A ; SEITZ E W.** Antimicrobial activity of aroma chemicals and essential oils. *Journal of the American Oil Chemistry Society,* 1979, vol. 56, 595-603
- **M DEVOS et al.** Standardized Human Olfactory Thresholds. ERL Press at Oxford University Press, 1990
- Compilation of Odor and Taste Threshold Values Data. ASTM Data Series DS 48A. 1978
- **O'TOOLE G. A. ; PRATT L. A. ; WATNICK P. L ; NEWMAN D. K. ; WEAVER V. B. ; KOLTER R.** Genetic approaches to the study ofbiofilms. *Methods in Enzymology,* 1999, vol. 310, 91-109